(19) Europäisches Patentamt
European Patent Office
Office européen des brevets

(11) Veröffentlichungsnummer : **0 096 002**
**B1**

(12) # EUROPÄISCHE PATENTSCHRIFT

(45) Veröffentlichungstag der Patentschrift : **29.07.87**

(51) Int. Cl.⁴ : **C 07 D239/52**, C 07 D239/42, C 07 D239/46, C 07 D251/16, A 01 N 47/36// C07C143/78

(21) Anmeldenummer : **83810212.7**

(22) Anmeldetag : **20.05.83**

(54) **N-Phenylsulfonyl-N'-pyrimidinyl- und -triazinylharnstoffe.**

(30) Priorität : **26.05.82 CH 3232/82**

(43) Veröffentlichungstag der Anmeldung : **07.12.83 Patentblatt 83/49**

(45) Bekanntmachung des Hinweises auf die Patenterteilung : **29.07.87 Patentblatt 87/31**

(84) Benannte Vertragsstaaten : **AT BE CH DE FR GB IT LI NL**

(56) Entgegenhaltungen :
**EP-A- 0 044 210**
**EP-A- 0 044 807**
**EP-A- 0 044 808**
**EP-A- 0 070 802**
**CHEMICAL ABSTRACTS, Band 63, 1965, Spalte 520h - Spalte 521a, Columbus Ohio (US)**
**Die Akte enthält technische Angaben, die nach dem Eingang der Anmeldung eingereicht wurden und die nicht in dieser Patentschrift enthalten sind.**

(73) Patentinhaber : **CIBA-GEIGY AG**
**Klybeckstrasse 141**
**CH-4002 Basel (CH)**

(72) Erfinder : **Schurter, Rolf, Dr.**
**Holzmattstrasse 45**
**CH-4102 Binningen (CH)**
Erfinder : **Föry, Werner, Dr.**
**Benkenstrasse 65**
**CH-4054 Basel (CH)**
Erfinder : **Meyer, Willy**
**Talweg 49**
**CH-4125 Riehen (CH)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 096 002 B1

Jouve, 18, rue St-Denis, 75001 Paris, France

**Beschreibung**

Die vorliegende Erfindung betrifft neue, herbizid wirksame und pflanzenwuchsregulierende N-Phenylsulfonyl-N'-pyrimidinyl- und -triazinyl-harnstoffe, Verfahren zu ihrer Herstellung, sie als Wirkstoffe enthaltende Mittel, sowie deren Verwendung zum Bekämpfen von Unkräutern, vor allem selektiv in Nutzpflanzenkulturen oder zum Regulieren und Hemmen des Pflanzenwachstums. Darüber hinaus betrifft die Erfindung auch als Zwischenprodukte hergestellte neue Phenylsulfonamide.

Die erfindungsgemässen N-Phenylsulfonyl-N'-pyrimidinyl- und triazinylharnstoffe entsprechen der allgemeinen Formel I

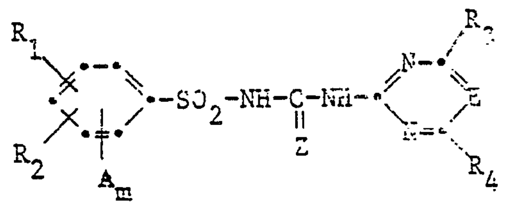

(I)

worin

A einen Rest der Formel $-C \equiv C-R$,

m die Zahl ein s oder zwei,

E die Methingruppe oder Stickstoff,

Z Sauerstoff oder Schwefel,

R Wasserstoff ; gegebenenfalls durch Halogen, Hydroxyl, $C_1$-$C_4$-Alkoxy, $C_1$-$C_4$-Alkylthio, $C_1$-$C_4$-Halogenalkoxy, $C_3$-$C_9$-Cycloalkyl, Cyan, $-COOR_6$, $-CONR_7R_8$ oder seinerseits gegebenenfalls durch Halogen, $C_1$-$C_4$-Alkyl, $C_1$-$C_4$-Alkoxy, Nitro, Cyan oder Trifluormethyl substituiertes Phenyl, substituiertes verzweigtes oder unverzweigtes $C_1$-$C_9$-Alkyl ; gegebenenfalls durch Halogen, Hydroxyl, $C_1$-$C_4$-Alkyl, $C_1$-$C_4$-Alkoxy, $C_1$-$C_4$-Alkylthio, Cyan, $-COOR_6$, $-CONR_7R_8$ substituiertes $C_3$-$C_9$-Cycloalkyl ; gegebenenfalls durch Halogen, Nitro, Cyan, $C_1$-$C_4$-Alkyl, $C_1$-$C_4$-Alkoxy, $C_1$-$C_4$-Alkylthio, Trifluormethyl, $-COOR_6$ oder $-CONR_7R_8$ substituiertes Phenyl ; oder einen gegebenenfalls durch Halogen, $C_1$-$C_4$-Alkyl, $C_1$-$C_4$-Alkoxy, $-COOR_6$ oder $-CONR_7R_8$ substituierten 5- bis 6-gliedrigen aromatischen Heterocyclus,

$R_1$ Wasserstoff, Halogen, $C_1$-$C_5$-Alkyl, $C_2$-$C_5$-Alkenyl oder einen Rest $-Y-R_5$,

$R_2$ Wasserstoff, Halogen, $C_1$-$C_5$-Alkyl, $C_2$-$C_5$-Alkenyl, $C_1$-$C_4$-Halogenalkyl, oder einen Rest $-Y-R_5$, $-COOR_6$, $-NO_2$ oder $-CO-NR_7-R_8$,

$R_3$ und $R_4$ unabhängig voneinander Wasserstoff, $C_1$-$C_4$-Alkyl, $C_1$-$C_4$-Alkoxy, $C_1$-$C_4$-Alkylthio, $C_1$-$C_4$-Halogenalkyl, $C_1$-$C_4$-Halogenalkoxy, $C_1$-$C_4$-Halogenalkylthio, Halogen, $C_2$-$C_5$-Alkoxyalkyl, $-NR_9R_{10}$ oder $-O-CH_2-CH_2-NR_9R_{10}$,

$R_5$ und $R_6$ unabhängig voneinander $C_1$-$C_5$-Alkyl, $C_2$-$C_5$-Alkenyl oder $C_2$-$C_6$-Alkinyl, $C_1$-$C_5$-Halogenalkyl, $C_2$-$C_5$-Halogenalkenyl oder $C_2$-$C_6$-Alkoxyalkyl,

$R_7$ und $R_8$ unabhängig voneinander Wasserstoff, $C_1$-$C_5$-Alkyl, $C_2$-$C_5$-Alkenyl oder $C_2$-$C_6$-Alkinyl,

$R_9$ Wasserstoff, Methyl oder Aethyl,

$R_{10}$ Wasserstoff, $C_1$-$C_4$-Alkyl, $C_1$-$C_4$-Alkoxy, Methoxymethyl, Cyanomethyl, Cyanoäthyl, $C_3$-$C_5$-Alkenyl oder $C_3$-$C_6$-Alkinyl und

Y Sauerstoff, Schwefel, eine Sulfinyl- oder Sulfonylbrücke bedeuten,

sowie den Salzen dieser Verbindungen.

Harnstoffverbindungen, Triazinverbindungen und Pyrimidinverbindungen mit herbizider Wirkung sind allgemein bekannt. Arylsulfamoyl-heterocyclyl-aminocarbamoylverbindungen mit herbizider und pflanzenwuchsregulierender Wirkung sind beispielsweise im niederländischen Patent Nr. 121 788 oder in den europäischen Patentpublikationen EP-A-44 210, EP-A-44 807, EP-A-44 808 und EP-A-70 802 beschrieben.

In den Definitionen ist unter Alkyl geradkettiges oder verzweigtes Alkyl zu verstehen ; z. B. : Methyl, Aethyl, n-Propyl, i-Propyl, die vier isomeren Butyl, n-Amyl, i-Amyl, 2-Amyl, 3-Amyl, n-Hexyl, i-Hexyl, oder die verschiedenen Heptyl-, Octyl- oder Nonylisomeren.

Unter Alkoxy ist zu verstehen : Methoxy, Aethoxy, n-Propyloxy, i-Propyloxy und die vier isomeren Butyloxyreste, insbesondere aber Methoxy, Aethoxy oder i-Propoxy.

Beispiele für Alkylthio sind Methylthio, Aethylthio, n-Propylthio, i-Propylthio und n-Butylthio, insbesondere aber Methylthio und Aethylthio.

Beispiele für Alkenylreste sind Vinyl, Allyl, Isopropenyl, 1-Propenyl, 1-Butenyl, 2-Butenyl, 3-Butenyl, 1-Isobutenyl, 2-Isobutenyl, 1-Pentenyl, 2-Pentenyl, 3-Pentenyl und 4-Pentenyl, insbesondere aber Vinyl, Allyl und 4-Pentenyl.

Unter Halogen in den Definitionen sowie in Halogenalkyl, -alkoxy, und -alkylthio sind Fluor, Chlor und Brom, vorzugsweise jedoch Fluor und Chlor zu verstehen.

Alkinylresten in den Definitionen der obigen Symbole entsprechen in der Regel Propargyl, 2-Butinyl,

3-Butinyl, sowie isomere Pentinyl- oder Hexinylreste, vorzugsweise ist der Alkinylrest jedoch durch Propargyl oder 2- oder 3-Butinyl verkörpert.

Beispiele für aromatische Heterocyclen, die Bestandteil des Substituenten A sein können, sind : Furan, Thiophen, Pyrrol, Pyrazol, Triazol, Pyridin, Pyrimidin, Pyridazin, Triazin, Thiazol, Oxazol, Thiadiazol oder Oxadiazol. Bevorzugt sind Furan, Thiophen und Pyridin. Ganz besonders bevorzugt sind diese Heterocyclen, wenn sie in der 2-Stellung mit der Aethinylbrücke verknüpft sind.

Die Erfindung umfasst ebenfalls die Salze, die die Verbindungen der Formel I mit Aminen, Alkali- und Erdalkalimetallbasen oder quaternären Ammoniumbasen bilden können.

Unter Alkali- und Erdalkalimetallhydroxiden als Salzbildner sind die Hydroxide von Lithium, Natrium, Kalium, Magnesium oder Calcium hervorzuheben, insbesondere aber die von Natrium oder Kalium.

Beispiele für zur Salzbildung geeignete Amine sind primäre, sekundäre und tertiäre aliphatische und aromatische Amine wie Methylamin, Aethylamin, Propylamin, i-Propylamin, die vier isomere Butylamine, Dimethylamin, Diäthylamin, Diäthanolamin, Dipropylamin, Diisopropylamin, Di-n-butylamin, Pyrrolidin, Piperidin, Morpholin, Trimethylamin, Triäthylamin, Tripropylamin, Chinuclidin, Pyridin, Chinolin und i-Chinolin, insbesondere aber Aethyl-, Propyl-, Diäthyl- oder Triäthylamin, vor allem aber iso-Propylamin und Diäthanolamin.

Beispiele für quaternäre Ammoniumbasen sind im allgemeinen die Kationen von Halogenammoniumsalzen, z. B. das Tetramethylammoniumkation, das Trimethylbenzylammoniumkation, das Triäthylbenzylammoniumkation, das Tetraäthylammoniumkation, das Trimethyläthylammoniumkation, aber auch das Ammoniumkation.

Von den erfindungsgemässen Verbindungen der Formel sind die Verbindungen bevorzugt, in denen

a) Z Sauerstoff bedeutet,
b) die Reste $R_3$ und $R_4$ zusammen nicht mehr als 4 Kohlenstoffatome enthalten und
c) m die Zahl eins bedeutet.

Aus der Gruppe c) sind die Verbindungen bevorzugt, in denen der Rest A in der 2- oder 3-Position zum Sulfonylrest steht.

Eine besonders bevorzugte Gruppe von Verbindungen der Formel I ist dadurch gekennzeichnet, dass in den Verbindungen nur ein Rest A in der 2- oder 3-Stellung zum Sulfonylrest vorhanden ist, Z Sauerstoff bedeutet und die Reste $R_3$ und $R_4$ zusammen nicht mehr als 4 Kohlenstoffatome enthalten.

Aus dieser Gruppe sind die Verbindungen bevorzugt, in denen $R_1$ Wasserstoff bedeutet und der Rest $R_2$ in der 5- oder 6-Stellung zur Sulfonylgruppe steht.

Von diesen Verbindungen sind wiederum die bevorzugt, in denen $R_2$ für Wasserstoff, Halogen, $C_1$-$C_4$-Alkoxy, $C_1$-$C_4$-Halogenalkoxy, Nitro, $C_1$-$C_4$-Alkyl, $C_1$-$C_4$-Halogenalkyl oder $COOR_6$ steht.

Eine weitere Bevorzugung innerhalb der letztgenannten Gruppe geniessen die Verbindungen, in denen $R_2$ Wasserstoff, Chlor, Fluor, $COOR_6$, Nitro, Methyl, Trifluormethyl, $C_1$-$C_4$-Halogenalkoxy oder $C_1$-$C_4$-Alkoxy und $R_3$ und $R_4$ jeweils Wasserstoff, $C_1$-$C_4$-Alkyl, $C_1$-$C_4$-Alkoxy, $C_1$-$C_4$-Alkylthio, Dialkylamino, $C_1$-$C_3$-Halogenalkoxy, Halogen oder Alkoxyalkyl bedeuten, wobei $R_3$ und $R_4$ zusammen höchstens 4 Kohlenstoffatome enthalten.

Von den genannten einzelnen bevorzugten Untergruppen von Verbindungen der Formel I, sind stets diejenigen bevorzugt hervorzuheben, bei denen R für Wasserstoff oder gegebenenfalls durch Halogen, Hydroxyl, Methoxy, Methylthio, $C_1$-$C_4$-Halogenalkoxy, Cyclopropyl, Cyan, Methoxycarbonyl substituiertes, verzweigtes oder unverzweigtes $C_1$-$C_4$-Alkyl, oder Phenyl, Pyridyl, Thienyl oder Furyl steht.

Als ganz besondere Untergruppe sind solche Verbindungen der Formel I hervorzuheben, in denen $R_1$ Wasserstoff, $R_2$ Wasserstoff, Chlor, Fluor, Nitro, Methyl, Trifluormethyl, $C_1$-$C_4$-Alkoxy, $C_1$-$C_4$-Halogenalkoxy oder $C_1$-$C_4$-Alkoxycarbonyl, das sich in 6-Position befindet, m die Zahl eins, $R_3$ und $R_4$ jeweils $C_1$-$C_4$-Alkyl, $C_1$-$C_4$-Alkoxy, Methylthio, $C_1$-$C_2$-Halogenalkoxy, Halogen oder Alkoxyalkyl, wobei $R_3$ und $R_4$ zusammen höchstens 4 Kohlenstoffatome enthalten, bedeuten, R für Wasserstoff oder gegebenenfalls durch Halogen, Hydroxyl, Methoxy, Methylthio, $C_1$-$C_4$-Halogenalkoxy, Cyclopropyl, Cyan, Methoxycarbonyl substituiertes, verzweigtes oder unverzweigtes $C_1$-$C_4$-Alkyl, oder Phenyl, Pyridyl, Thienyl oder Furyl steht.

Als bevorzugte Einzelverbindungen sind zu nennen :

N-[2-(1-Propinyl)-phenylsulfonyl]-N'-(4,6-dimethoxypyrimidin-2-yl)-harnstoff,
N-[2-(1-Propinyl)-phenylsulfonyl]-N'-(4-methoxy-6-methylpyrimidin-2-yl)-harnstoff,
N-[2-(1-Propinyl)-phenylsulfonyl]-N'-(4,6-dimethyl-pyrimidin-2-yl)-harnstoff,
N-[2-(2-Phenyläthinyl)-phenylsulfonyl]-N'-(4,6-dimethoxypyrimidin-2-yl)-harnstoff,
N-[2-(1-Propinyl)-phenylsulfonyl]-N'-(4-methoxy-6-methyl-1,3,5-triazin-2-yl)-harnstoff und
N-[2-(3-Hydroxy-3'-methyl-butin-1'-yl)-phenylsulfonyl]-N'-(4,6-dimethoxy-pyrimidin-2-yl)-harnstoff.

Die Herstellung der Verbindungen der Formel I erfolgt in einem inerten, organischen Lösungsmittel.

Nach einem ersten Verfahren werden die Verbindungen der Formel I erhalten, indem man ein Phenylsulfonamid der Formel II

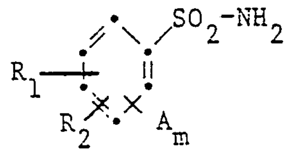

(II)

worin A, $R_1$, $R_2$ und m die unter Formel I gegebene Bedeutung haben, in Gegenwart einer Base mit einem N-Pyrimidinyl- oder -Triazinylcarbamat der Formel III

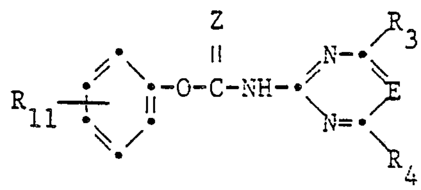

(III)

worin E, $R_3$, $R_4$ und Z die unter Formel I gegebene Bedeutung haben und $R_{11}$ für Wasserstoff, Halogen, $C_1$-$C_4$-Alkyl, $C_1$-$C_4$-Alkoxy, Nitro, Cyan oder Trifluormethyl steht, umsetzt.

Nach einem zweiten Verfahren gelangt man zu Verbindungen der Formel I, indem man ein Phenylsulfonylisocyanat oder -isothiocyanat der Formel IV

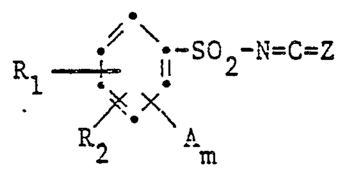

(IV)

worin A, $R_1$, $R_2$, m und Z die unter Formel I gegebene Bedeutung haben, gegebenenfalls in Gegenwart einer Base, mit einem Amin der Formel V

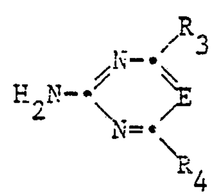

(V)

worin E, $R_3$ und $R_4$ die unter Formel I gegebene Bedeutung haben, umsetzt.

Nach einem weiteren Verfahren werden die Verbindungen der Formel I hergestellt, indem man ein Sulfonamid der oben angegebenen Formel II gegebenenfalls in Gegenwart einer Base mit einem Isocyanat oder Isothiocyanat der Formel VI

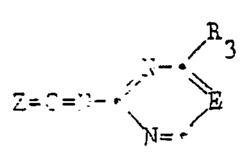

(VI)

worin E, $R_3$, $R_4$ und Z die unter Formel I gegebene Bedeutung haben, umsetzt.

Schliesslich kann man die Verbindungen der Formel I auch erhalten, indem man ein N-Phenylsulfonylcarbamat der Formel VII

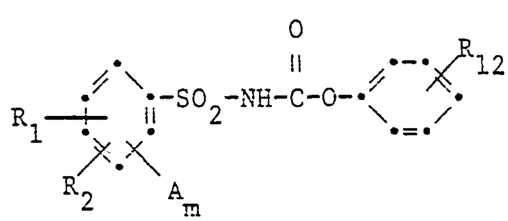

(VII)

worin A, $R_1$, $R_2$ und m die unter Formel I gegebene Bedeutung haben und $R_{12}$ für Wasserstoff, Halogen, $C_1$-$C_4$-Alkyl, $C_1$-$C_4$-Alkoxy, Nitro, Cyan oder Trifluormethyl steht, mit einem Amin der oben angegebenen Formel V umsetzt.

Die erhaltenen Harnstoffe der Formel I können gewünschtenfalls mittels Aminen, Alkalimetall- oder Erdalkalimetallhydroxiden oder quaternären Ammoniumbasen in Additionssalze übergeführt werden. Dieses geschieht beispielsweise durch Umsetzen mit der äquimolaren Menge Base und Verdampfen des Lösungsmittels.

Die Ausgangsstoffe der Formel II, IV und VII sind neu und können nach folgenden Methoden hergestellt werden :

Die neuen und als Zwischenprodukte verwendeten Sulfonamide der Formel II werden aus den entsprechenden Halogenphenylsulfonamiden (IX) durch Umsetzung mit Acetylenverbindungen gemäss dem folgenden Schema A erhalten. Analoge Darstellungen von Aethinylverbindungen sind in der europäischen Patentanmeldung Nr. 41476 beschrieben.

(Siehe Schema Seite 6 f.)

Im Schema A haben R, $R_1$ und $R_2$ die unter Formel I angegebene Bedeutung mit der Massgabe, dass R in der Formel XI für einen aromatischen Rest steht. Unter Hal ist Chlor, insbesondere aber Brom und Jod zu verstehen. Alkyl steht für einen $C_1$-$C_4$-Alkylrest.

Methode α ist ein Verfahren, das es erlaubt, unter Verwendung von Metallkatalysatoren, halogenierte Reste, wie in den Formeln IX oder XI, mit endständigen Acetylengruppen, wie in den Formeln VIII, X und XIII, unter milden Reaktionsbedingungen in Gegenwart eines säurebindenden Mittels zu verbinden. Reaktionen dieser Art sind in folgenden Literaturstellen beschrieben : K. Sonogarshira, Y. Rohda und N. Hagihara, Tetrahedron Lett., 50, 4467 (1975) ; L. Cassar, J. Organomet. Chem., 93, 253 (1975) und H.A. Dieck und F.R. Heck, J. Organomet. Chem., 93, 259 (1975).

Diese Reaktion findet vorteilhafterweise in gegenüber den Reaktionspartnern inerten, organischen Lösungsmitteln statt. Als solche kommen viele protische wie auch aprotische Lösungsmittel in Frage, z. B. Alkohole, Ketone, Aether, Kohlenwasserstoffe, halogenierte Kohlenwasserstoffe, aromatische Lösungsmittel wie Methanol, Aethanol, Isopropanol, Cyclohexanon, Aceton, Methyläthylketon, Diäthyläther, Dimethyläther, Tetrahydrofuran, Dioxan, Cyclohexan, Pentan, Hexan, Heptan, Octan, Methylenchlorid, Chloroform, Tetrachlorkohlenstoff, Benzol, Toluol oder Xylol oder auch z. B. Dimethylformamid, Dimethylsulfoxid, Acetonitril oder tertiäre Amine wie Triäthylamin.

Als säurebindendes Mittel kann, da bei der Umsetzung Halogenwasserstoff abgespalten wird, eine Base eingesetzt werden. Dafür kommen starke anorganische Basen wie KOH, NaOH in Frage, aber auch organische wie beispielsweise Triäthylamin, Diäthylamin, Pyridin, Alkoholate etc. Bei den Reaktionen werden gegebenenfalls 1-5 Aequivalente dieser Basen eingesetzt.

Als Metallkatalysatoren werden vorzugsweise Palladiumsalze oder -komplexe verwendet, insbesondere Palladiumchlorid $PdCl_2$, Palladiumacetat $Pd(OCOCH_3)_2$ oder der Palladiumdichlor-bis-(triphenylphosphin)-Komplex $PdCl_2[P(C_6H_5)_3]_2$, meist unter Zusatz eines Kupfer-(I)-Salzes, insbesondere von Kupfer-(I)-jodid. Die Katalysatoren werden als solche oder aufgezogen auf einem Träger wie z. B. Kohlepulver, Aluminiumoxid etc., verwendet.

Die Reaktionstemperaturen liegen in der Regel zwischen 0° und 200 °C, vorwiegend jedoch zwischen Raumtemperatur und der Siedetemperatur des Reaktionsgemisches.

Die Reaktionszeiten liegen im allgemeinen zwischen 0,5 und 48 Stunden.

Die Methode β ermöglicht es, in Gegenwart einer starken Base wie NaOH, KOH oder Alkoholat aus einem tertiären Aethinylalkohol, wie in den Formeln X, XII und XIV, welcher als geschützte endständige Acetylengruppe aufgefasst werden kann, unter Abspaltung der Ketoschutzgruppe das Acetylen, wie in den Formeln VIII und XIII, freizusetzen. Die als Nebenprodukte entstehenden Ketone können aus dem Reaktionsgemisch während der Reaktion destillativ entfernt werden. Reaktionen dieser Art sind in der DOS 2 905 507 und im US-Patent 4 128 588 beschrieben.

Diese Reaktion wird vorteilhafterweise in gegenüber den Reaktionspartnern inerten, organischen Lösungsmitteln wie Alkoholen, Aethern, Ketonen, Kohlenwasserstoffen, Halogenkohlenwasserstoffen, aromatischen Lösungsmitteln oder auch Dimethylformamid, Dimethylsulfoxid oder Acetonitril durchgeführt. Beispiele für solche Lösungsmittel sind : Methanol, Aethanol, Isopropanol, Dimethyläther, Tetrahydrofuran, Dioxan, Aceton, Methyläthylketon, Cyclohexanon, Cyclohexan, Benzol, Toluol, Xylol, Methylenchlorid, Chloroform oder Tetrachlorkohlenstoff.

Die Reaktionstemperatur liegt auch hier vorzugsweise zwischen Raumtemperatur und dem Siedepunkt des Reaktionsgemisches.

Die Reaktionszeit beträgt im allgemeinen zwischen 0,5 und 12 Stunden.

Die Methode γ besteht aus einer Kombination der Methoden α und β, wobei das nach der Methode α umzusetzende Acetylen in situ durch Einwirkung einer starken Base auf ein geschütztes Acetylen der Formel X, XII und XIV hergestellt wird.

Die Reaktionsbedingungen sind mit denen der Methode α identisch.

Zwingend erforderlich ist jedoch der Zusatz einer starken Base wie NaOH, KOH oder das Alkalisalz

Schema A

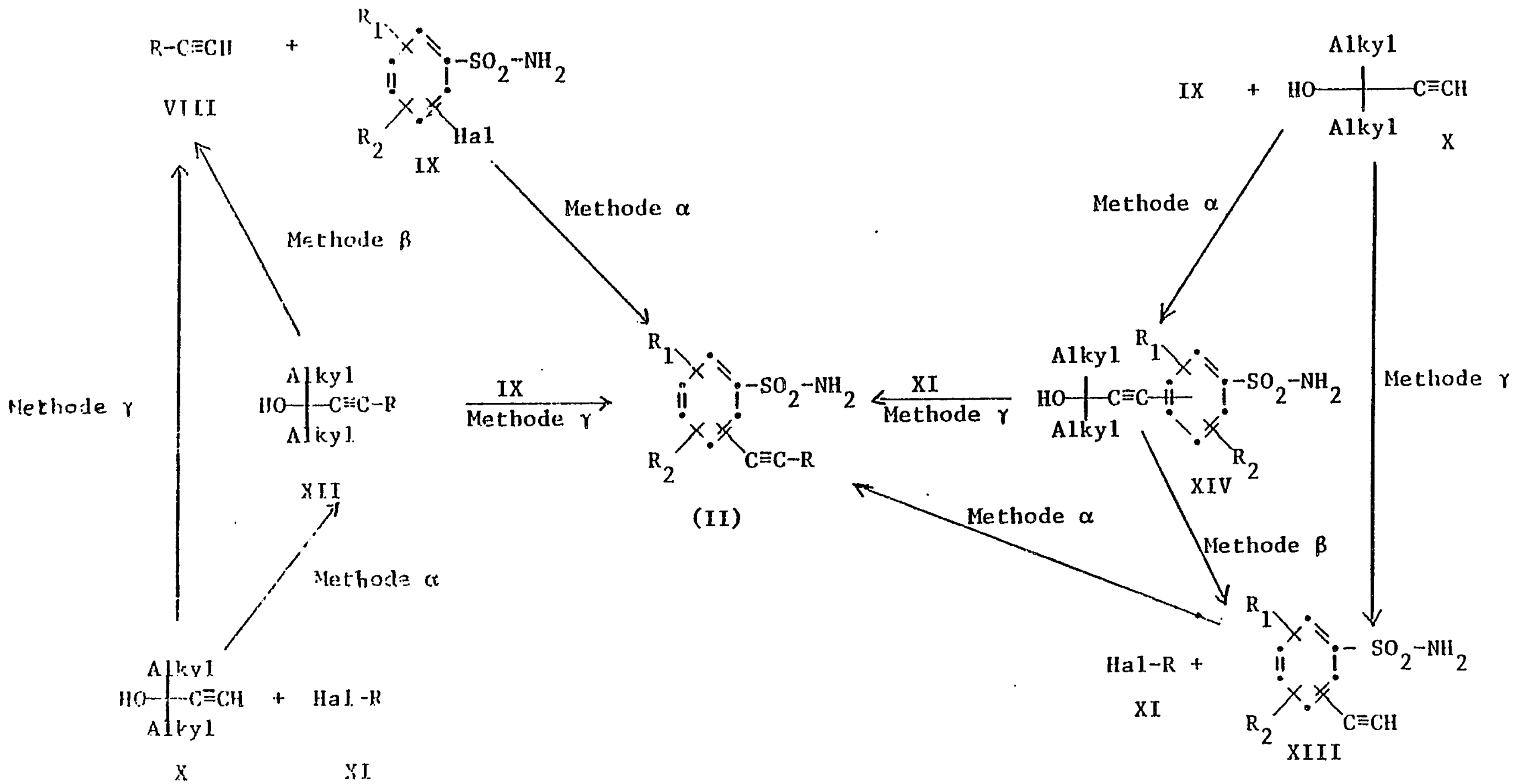


(In der Formel XI bedeutet R einen aromatischen Rest)

eines Alkohols.

Nach dem oben skizzierten Verfahren werden die Verbindungen der Formel II erhalten, indem man entweder eine Aethinylverbindung der Formel VIII

$$R—C{\equiv}CH \quad (VIII)$$

in Gegenwart eines säurebindenden Mittels und eines Metallkatalysators gegebenenfalls unter einer Inertgasatmosphäre mit einem Halogenphenylsulfonamid der Formel IX

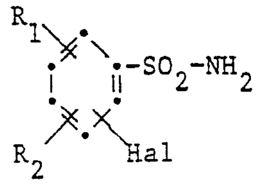

(IX)

umsetzt oder indem man ein aromatisches Halogenid der Formel XI

$$Hal—R' \quad (XI)$$

unter den gleichen Reaktionsbedingungen mit einem Aethinylphenylsulfonamid der Formel XIII

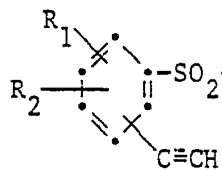

(XIII)

worin $R_1$ und $R_2$ die unter Formel I gegebene Bedeutung haben, R' für einen aromatischen Rest steht und Hal Brom oder Jod ist, umsetzt.

Nach einem weiteren Verfahren werden die Verbindungen der Formel II erhalten, indem man ein aromatisches Halogenid der Formel XI

$$Hal—R' \quad (XI)$$

in Gegenwart eines säurebindenden Mittels und eines Metallkatalysators gegebenenfalls unter einer Inertgasatmosphäre mit einem Propargylalkohol der Formel X

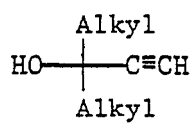

(X)

umsetzt und die erhaltene Aethinylverbindung der Formel XII

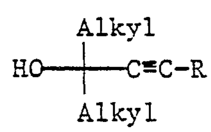

(XII)

in Gegenwart einer starken Base und eines Metallkatalysators gegebenenfalls unter einer Inertgasatmosphäre mit einem Halogenphenylsulfonamid der Formel IX

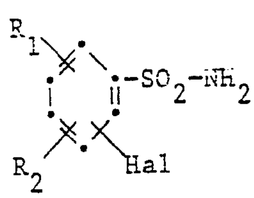

(IX)

umsetzt oder indem man das Halogenphenylsulfonamid der Formel IX unter den obigen Bedingungen zuerst mit dem Propargylalkohol der Formel X umsetzt und dann die entstandene Aethinylverbindung der Formel XIV

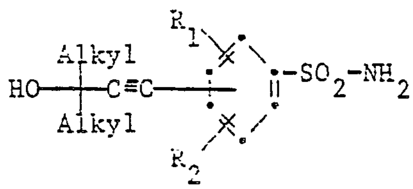

unter den obigen Bedingungen mit dem aromatischen Halogenid der Formel XI, worin $R_1$ und $R_2$ die unter Formel I gegebene Bedeutung haben, Hal Brom oder Jod ist und Alkyl ein $C_1$-$C_4$-Alkylrest ist, umsetzt.

Die als Zwischenprodukte verwendeten Verbindungen der Formel II sind neu und speziell für die Synthese von Verbindungen der Formel I entwickelt worden. Diese Zwischenprodukte bilden einen weiteren Aspekt der vorliegenden Erfindung.

Die Phenylsulfonylisocyanate der Formel IV können durch Umsetzungen der Phenylsulfonamide der Formel II mit Phosgen in Anwesenheit von Butylisocyanat in einem chlorierten Kohlenwasserstoff als Lösungsmittel, bei Rückflusstemperatur erhalten werden. Aehnliche Darstellungen sind in « Neuere Methoden der präparativen organischen Chemie », Band VI, 211-229, Verlag Chemie, Weinheim, 1970, beschrieben.

Die Isothiocyanate der Formel IV werden durch Behandlung der Phenylsulfonamide der Formel II mit Schwefelkohlenstoff und Kaliumhydroxid und anschliessender Umsetzung des Dikaliumsalzes mit Phosgen erhalten. Solche Verfahren sind in Arch. Pharm. 299, 174 (1966) beschrieben.

Die N-Phenylsulfonylcarbamate der Formel VII werden durch Umsetzung der Phenylsulfonamide der Formel II mit Diphenylcarbonat in Gegenwart einer Base erhalten. Aehnliche Verfahren sind in der japanischen Patentschrift 61 169 erwähnt.

Die Ausgangsmaterialien der Formeln III, V und VI sind bekannt oder können nach bekannten Methoden hergestellt werden.

Durch Umsetzung von heterocyclischen Aminen der Formel V mit Oxalylchlorid in chlorierten Kohlenwasserstoffen als Lösungsmittel, lassen sich Isocyanate der Formel VI herstellen, Amine der Formel V sind bekannt und zum Teil im Handel erhältlich oder sie können nach bekannten Methoden hergestellt werden, siehe « The Chemistry of Heterocyclic Compounds », Band XIV, Interscience Publishers, New York, London.

Diese Umsetzungen zu Verbindungen der Formel I werden vorteilhafterweise in aprotischen, inerten, organischen Lösungsmitteln vorgenommen wie Methylenchlorid, Tetrahydrofuran, Acetonitril, Dioxan, Toluol.

Die Reaktionstemperaturen liegen vorzugsweise zwischen — 20° und + 120 °C. Die Umsetzungen verlaufen im allgemeinen leicht exotherm und können bei Raumtemperatur durchgeführt werden. Zwecks Abkürzung der Reaktionszeit oder auch zum Einleiten der Umsetzung wird zweckdienlich für kurze Zeit bis zum Siedepunkt des Reaktionsgemisches aufgewärmt. Die Reaktionszeiten können ebenfalls durch Zugabe einiger Tropfen Base oder Isocyanat als Reaktionskatalysator verkürzt werden.

Die Endprodukte können durch Einengen und/oder Verdampfen des Lösungsmittels isoliert und durch Umkristallisieren oder Zerreiben des festen Rückstandes in Lösungsmitteln in denen sie sich nicht gut lösen, wie Aether, aromatischen Kohlenwasserstoffen oder chlorierten Kohlenwasserstoffen gereinigt werden.

Die Wirkstoffe der Formel I sind stabile Verbindungen. Ihre Handhabung bedarf keiner vorsorglicher Massnahmen.

Bei geringeren Aufwandmengen zeichnen sich die Verbindungen der Formel I durch gute selektivwuchshemmende und selektiv-herbizide Eigenschaften aus, die sie ausgezeichnet zum Einsatz in Kulturen von Nutzpflanzen, insbesondere in Getreide, Baumwolle, Soja, Mais und Reis, befähigen. Es werden dabei teilweise auch Unkräuter geschädigt, welchen bisher nur mit Totalherbiziden beizukommen war.

Die Wirkungsart einiger dieser Wirkstoffe ist unüblich. Viele sind transolzierbar, d. h. sie werden von der Pflanze aufgenommen und an andere Stellen transportiert, wo sie dann zur Wirkung kommen. So gelingt es beispielsweise, durch Oberflächenbehandlung perennierende Unkräuter bis in die Wurzeln zu schädigen. Die neuen Verbindungen der Formel I wirken bereits bei — im Vergleich zu anderen Herbiziden und Wuchsregulatoren — sehr geringen Aufwandmengen.

Die Verbindungen der Formel I haben ausserdem starke pflanzenwuchsregulierende, insbesondere pflanzenwuchshemmende, Eigenschaften. Es werden sowohl Monokotyledonen als auch Dikotyledonen in ihrem Wachstum beeinträchtigt.

So können z. B. die in der Landwirtschaft in tropischen Gegenden häufig als « cover crops » (Bodenbedecker) angepflanzten Leguminosen durch die Verbindungen der Formel I in ihrem Wachstum

selektiv gehemmt werden, so dass zwar die Bodenerosion zwischen den Kulturpflanzen verhindert wird, die « cover crops » jedoch nicht zur Konkurrenz für die Kultur werden können.

Weiter eignen sich die Verbindungen der Formel I um das Keimen von eingelagerten Kartoffeln zu verhindern. Bei Kartoffeln entwickeln sich bei der Einlagerung über den Winter häufig Keime, die Schrumpfen, Gewichtsverlust und Faulen zur Folge haben.

Bei grösseren Aufwandmengen werden alle getesteten Pflanzen in ihrer Entwicklung so geschädigt, dass sie absterben.

Die Erfindung betrifft auch herbizide und pflanzenwachstumsregulierende Mittel, welche einen neuen Wirkstoff der Formel I enthalten, sowie Verfahren zur pre- und post-emergenten Unkrautbekämpfung und zur Hemmung des Pflanzenwuchses von monokotylen und dikotylen Pflanzen, insbesondere Gräsern, tropischen Bodenbedeckern und Tabakgeiztrieben.

Die Verbindungen der Formel I werden in unveränderter Form oder vorzugsweise als Mittel zusammen mit den in der Formulierungstechnik üblichen Hilfsmitteln eingesetzt und werden daher z. B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z. B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d. h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden).

Als Lösungsmittel können in Frage kommen : Aromatische Kohlenwasserstoffe, bevorzugt die Fraktionen $C_8$ bis $C_{12}$, wie z. B. Xylolgemische oder substituierte Naphthaline, Phthalsäureester wie Dibutyl- oder Dicotylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl ; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kielselsäure oder hochdisperse saugfähige Polymerisate zugesetzt werden. Als gekörnte, adsorptive Granulatträger kommen poröse Typen wie z. B. Bimsstein, Ziegelbruch, Sepiolit oder Bentonit, als nicht sorptive Trägermaterialien z. B. Calcit oder Sand in Frage. Darüberhinaus kann eine Vielzahl von vorganulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z. B. die Na- oder K-Salze der Oel- oder Stearinsäure, oder von natürlichen Fettsäuregemischen, die z. B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z. B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8-22 C-Atomen. Alkylarylsulfonate sind z. B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z. B. Salze des Phosphorsäureesters eines p-Nonylphenol-(4-14)-Aethylenoxid-Adduktes oder Phospholipide in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitantrioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl oder niedrige Hydroxyalkylreste aufweisen. Die Salze liegen vorzugsweise als Halogenide, Methylsulfate oder Aethylsulfate vor, z. B. das Stearyltrimethylammoniumchlorid oder das Benzyldi (2-chloräthyl)-äthylammoniumbromid.

Die in der Formulierungstechnik gebräuchlichen Tenside sind u. a. in folgenden Publikationen beschrieben : « Mc Cutcheon's Detergents and Emulsifiers Annual » MC Publishing Corp., Ridgewood, New Jersey, 1979. Sisley and Wood, « Encyclopedia of Surface Active Agents ». Chemical Publishing Co., Inc. New York, 1964.

Die pestiziden Zubereitungen enthalten in der Regel 0,1 bis 95 %, insbesondere 0,1 bis 80 %, Wirkstoff der Formel I, 1 bis 99,9 % eines festen oder flüssigen Zusatzstoffes und 0 bis 25 %, insbesondere 0,1 bis 25 % eines Tensides.

Insbesondere setzen sich bevorzugte Formulierungen folgendermassen zusammen : (% = Gewichtsprozent)

| | |
|---|---|
| Emulgierbare Konzentrate | |
| Aktiver Wirkstoff : | 1 bis 20 %, bevorzugt 5 bis 10 % |
| oberflächenaktives Mittel : | 5 bis 30 %, vorzugsweise 10 bis 20 % |
| flüssiges Trägermittel : | 50 bis 94 %, vorzugsweise 70 bis 85 %. |
| Stäube | |
| Aktiver Wirkstoff : | 0,1 bis 10 %, vorzugsweise 0,1 bis 1 % |
| festes Trägermittel : | 99,9 bis 90 %, vorzugsweise 99,9 bis 99 %. |
| Suspension-Konzentrate | |
| Aktiver Wirkstoff : | 5 bis 75 %, vorzugsweise 10 bis 50 % |
| Wasser : | 94 bis 25 %, vorzugsweise 90 bis 30 % |
| oberflächenaktives Mittel : | 1 bis 40 %, vorzugsweise 2 bis 30 %. |
| Benetzbare Pulver | |
| Aktiver Wirkstoff : | 0,5 bis 90 %, vorzugsweise 1 bis 80 % |
| oberflächenaktives Mittel : | 0,5 bis 20 %, vorzugsweise 1 bis 15 % |
| festes Trägermittel : | 5 bis 95 %, vorzugsweise 15 bis 90 %. |
| Granulate | |
| Aktiver Wirkstoff : | 0,5 bis 30 %, vorzugsweise 3 bis 15 % |
| festes Trägermittel : | 99,5 bis 70 %, vorzugsweise 97 bis 85 %. |

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,01 bis 10 kg AS/ha, vorzugsweise 0,025 bis 5 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

In den folgenden Beispielen sind die Temperaturen in Celsiusgraden °C angegeben.

## Herstellungsbeispiele

### Beispiel 1

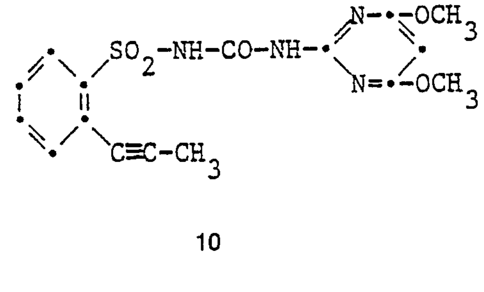

N-[2(1-Propinyl)-phenyl-sulfonyl]-N'-(4,6-dimethoxy-pyrimidin-2-yl)-harnstoff (Verbindung 5.1)

a) 2-(1-Propinyl)-phenylsulfonamid

Eine Lösung von 28,3 g (0,1 Mol) 2-Jod-phenylsulfonamid in 350 ml Dimethylformamid und 100 ml Triäthylamin wird mit 1,0 g Palladiumdichlor-bis-(triphenylphosphin)-komplex $PdCl_2[P(C_6H_5)_3]_2$ und 0,5 g Kupfer-(I)-jodid CuJ versetzt. In diese Lösung leitet man gasförmiges Propin ein, bis das Ausgangsmaterial verbraucht ist. Nach Filtration und Eindampfen im Vakuum wird der Rückstand mit Wasser versetzt, der entstandene Niederschlag abgetrennt und getrocknet. Durch Umkristallisieren aus Aethylacetat/Hexan erhält man 15,6 g (80 %) 2-(1-Propinyl)-phenylsulfonamid, Smp. 147-149 °C.

b) N-[2-(1-Propinyl)-phenylsulfonyl]-N'-4,6-dimethoxy-pyrimidin-2-yl)-harnstoff

Einer Lösung von 1,95 g (1,01 Mol) 2-(1-Propinyl)-phenylsulfonamid und 1,7 g 1,8-Diazabicyclo [5.4.0]-undec-7-en in 33 ml absolutem Dioxan setzt man portionsweise innerhalb von 30 Minuten bei 20-25 °C 2,75 g (1,01 Mol) 4,6-Dimethoxy-2-phenoxycarbonylamino-pyrimidin zu. Nachdem die Reaktionsmischung für 4 Stunden bei 20-25 °C gerührt worden ist, wird sie in einem Gemisch von 66 ml Eiswasser, 10 ml 2N-Salzsäure und 150 ml Aethylacetat aufgenommen. Die organische Phase wird abgetrennt, die wässrige nochmals mit 66 ml Aethylacetat extrahiert. Die vereinigten organischen Extrakte werden mit Wasser und gesättigter Kochsalzlösung gewaschen, über Magnesiumsulfat getrocknet und eingedampft. Der kristalline Rückstand wird mit Diäthyläther gewaschen. Man erhält so 2,4 g (74 %) N-[2-(1-Propinyl)-phenyl-sulfonyl]-N'-4,6-dimethoxy-pyrimidin-2-yl)-harnstoff, Smp. 193-194 °C.

In analoger Weise erhält man die in den anschliessenden Tabellen aufgelisteten Zwischen- und Endprodukte.

(Siehe Tabellen Seite 12 ff.)

Tabelle 1

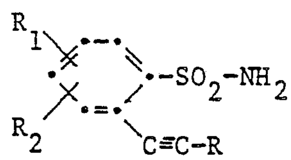

| Nr. | R | $R_2$ | $R_1$ | Smp. [°C] |
|---|---|---|---|---|
| 1.1 | $CH_3$ | H | H | 147-149 |
| 1.2 | H | H | H | |
| 1.3 | $-C(CH_3)_2-OH$ | H | H | 200-201 |
| 1.4 | $C_6H_5$ | H | H | 85-88 |
| 1.5 | $4-Cl-C_6H_4-$ | H | H | |
| 1.6 | 2-Thienyl | H | H | |
| 1.7 | 2-Furyl | H | H | |
| 1.8 | 2-Pyridyl | H | H | |
| 1.9 | $CH_3$ | 5-F | H | |
| 1.10 | H | 5-F | H | |
| 1.11 | $-C(CH_3)_2-OH$ | 5-F | H | |
| 1.12 | $C_6H_5$ | 5-F | H | |
| 1.13 | 2-Thienyl | 5-F | H | |
| 1.14 | 2-Furyl | 5-F | H | |
| 1.15 | $CH_3$ | 6-Cl | H | |
| 1.16 | $CH_3$ | $6-COOCH_3$ | H | |
| 1.17 | $CH_3$ | $6-OCH_3$ | H | |
| 1.18 | $CH_3$ | $6-OCHF_2$ | H | |
| 1.19 | $CH_3$ | $6-CF_3$ | H | |
| 1.20 | $CH_3$ | $6-CH_3$ | H | |

Tabelle 2

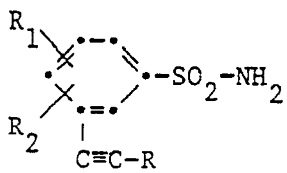

| Nr. | R | $R_2$ | $R_1$ | Smp. [°C] |
|---|---|---|---|---|
| 2.1 | $CH_3$ | 6-Cl | H | |
| 2.2 | H | 6-Cl | H | |
| 2.3 | $-C(CH_3)_2-OH$ | 6-Cl | H | |
| 2.4 | $C_6H_5$ | 6-Cl | H | |
| 2.5 | $CH_3$ | 6-F | H | |
| 2.6 | H | 6-F | H | |
| 2.7 | $-C(CH_3)_2-OH$ | 6-F | H | |
| 2.8 | $C_6H_5$ | 6-F | H | |
| 2.9 | $CH_3$ | H | H | |
| 2.10 | H | H | H | |
| 2.11 | $CH_3$ | $6-COOCH_3$ | H | |
| 2.12 | $CH_3$ | $6-CF_3$ | H | |
| 2.13 | $CH_3$ | $6-OCH_3$ | H | |
| 2.14 | $CH_3$ | $6-OCHF_2$ | H | |
| 2.15 | $CH_3$ | $6-CH_3$ | H | |

Tabelle 3

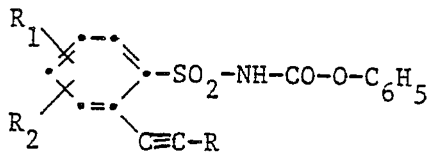

| Nr. | R | $R_2$ | $R_1$ | Smp. [°C] |
|---|---|---|---|---|
| 3.1 | $CH_3$ | H | H | |
| 3.2 | H | H | H | |
| 3.3 | $-C(CH_3)_2-OH$ | H | H | |
| 3.4 | $C_6H_5$ | H | H | |
| 3.5 | $4-Cl-C_6H_4-$ | H | H | |
| 3.6 | 2-Thienyl | H | H | |
| 3.7 | 2-Furyl | H | H | |
| 3.8 | 2-Pyridyl | H | H | |
| 3.9 | $CH_3$ | 5-F | H | |
| 3.10 | H | 5-F | H | |
| 3.11 | $-C(CH_3)_2-OH$ | 5-F | H | |
| 3.12 | $C_6H_5$ | 5-F | H | |
| 3.13 | 2-Thienyl | 5-F | H | |
| 3.14 | 2-Furyl | 5-F | H | |

Tabelle 4

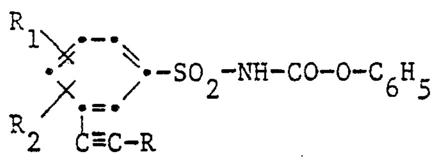

| Nr. | R | $R_2$ | $R_1$ | Smp. [°C] |
|---|---|---|---|---|
| 4.1 | $CH_3$ | 6-Cl | H | |
| 4.2 | H | 6-Cl | H | |
| 4.3 | $-C(CH_3)_2-OH$ | 6-Cl | H | |
| 4.4 | $C_6H_5$ | 6-Cl | H | |
| 4.5 | $CH_3$ | 6-F | H | |
| 4.6 | H | 6-F | H | |
| 4.7 | $-C(CH_3)_2-OH$ | 6-F | H | |
| 4.8 | $C_6H_5$ | 6-F | H | |

Tabelle 5

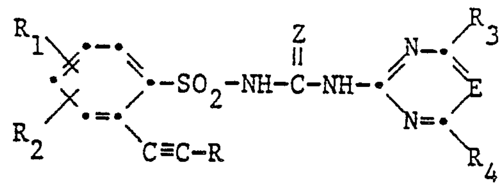

| Nr. | R | $R_2$ | $R_1$ | $R_3$ | $R_4$ | E | Z | Smp. [°C] |
|---|---|---|---|---|---|---|---|---|
| 5.1 | $CH_3$ | H | H | $OCH_3$ | $OCH_3$ | CH | O | 193-194 |
| 5.2 | $CH_3$ | H | H | $CH_3$ | $CH_3$ | CH | O | 187-189 |
| 5.3 | $CH_3$ | H | H | $CH_3$ | $OCH_3$ | CH | O | 193 (Zers.) |
| 5.4 | $CH_3$ | H | H | $OCH_3$ | $OCH_3$ | N | O | |
| 5.5 | $CH_3$ | H | H | $CH_3$ | $OCH_3$ | N | O | 168-170 |
| 5.6 | $CH_3$ | H | H | $OCH_3$ | $-N(CH_3)_2$ | N | O | |
| 5.7 | $CH_3$ | H | H | $CH_3$ | $-OCHF_2$ | CH | O | |
| 5.8 | $CH_3$ | H | H | $OCH_3$ | $-OCH_2-CF_3$ | N | O | |
| 5.9 | H | H | H | $OCH_3$ | $OCH_3$ | CH | O | |
| 5.10 | H | H | H | $CH_3$ | $CH_3$ | CH | O | |
| 5.11 | H | H | H | $CH_3$ | $OCH_3$ | CH | O | |
| 5.12 | H | H | H | $OCH_3$ | $OCH_3$ | N | O | |
| 5.13 | H | H | H | $CH_3$ | $OCH_3$ | N | O | |
| 5.14 | H | H | H | $OCH_3$ | $-N(CH_3)_2$ | N | O | |
| 5.15 | H | H | H | $CH_3$ | $-OCHF_2$ | CH | O | |
| 5.16 | H | H | H | $OCH_3$ | $-OCH_2-CF_3$ | N | O | |
| 5.17 | $C_6H_5$ | H | H | $OCH_3$ | $OCH_3$ | CH | O | 166-169 |
| 5.18 | $C_6H_5$ | H | H | $CH_3$ | $CH_3$ | CH | O | |
| 5.19 | $C_6H_5$ | H | H | $CH_3$ | $OCH_3$ | CH | O | |
| 5.20 | $C_6H_5$ | H | H | $OCH_3$ | $OCH_3$ | N | O | |
| 5.21 | $C_6H_5$ | H | H | $CH_3$ | $OCH_3$ | N | O | |
| 5.22 | $C_6H_5$ | H | H | $OCH_3$ | $-N(CH_3)_2$ | N | O | |
| 5.23 | $C_6H_5$ | H | H | $CH_3$ | $-OCHF_2$ | CH | O | |
| 5.24 | $C_6H_5$ | H | H | $OCH_3$ | $-OCH_2-CF_3$ | N | O | |
| 5.25 | $4-Cl-C_6H_4-$ | H | H | $OCH_3$ | $OCH_3$ | CH | O | |
| 5.26 | $4-Cl-C_6H_4-$ | H | H | $CH_3$ | $CH_3$ | CH | O | |

Tabelle 5 (Fortsetzung)

| Nr. | R | $R_2$ | $R_1$ | $R_3$ | $R_4$ | E | Z | Smp. [°C] |
|---|---|---|---|---|---|---|---|---|
| 5.27 | $4-Cl-C_6H_4-$ | H | H | $CH_3$ | $OCH_3$ | CH | O | |
| 5.28 | $4-Cl-C_6H_4-$ | H | H | $OCH_3$ | $OCH_3$ | N | O | |
| 5.29 | $4-Cl-C_6H_4$ | H | H | $CH_3$ | $OCH_3$ | N | O | |
| 5.30 | $4-Cl-C_6H_4-$ | H | H | $OCH_3$ | $-N(CH_3)_2$ | N | O | |
| 5.31 | $4-Cl-C_6H_4-$ | H | H | $CH_3$ | $-OCHF_2$ | CH | O | |
| 5.32 | $4-Cl-C_6H_4-$ | H | H | $OCH_3$ | $-OCH_2-CF_3$ | N | O | |
| 5.33 | $-C(CH_3)_2-OH$ | H | H | $OCH_3$ | $OCH_3$ | CH | O | 150 |
| 5.34 | $-C(CH_3)_2-OH$ | H | H | $CH_3$ | $CH_3$ | CH | O | |
| 5.35 | $-C(CH_3)_2-OH$ | H | H | $CH_3$ | $OCH_3$ | CH | O | |
| 5.36 | $-C(CH_3)_2-OH$ | H | H | $OCH_3$ | $OCH_3$ | N | O | |
| 5.37 | $-C(CH_3)_2-OH$ | H | H | $CH_3$ | $OCH_3$ | N | O | |
| 5.38 | $-C(CH_3)_2-OH$ | H | H | $OCH_3$ | $-N(CH_3)_2$ | N | O | |
| 5.39 | $-C(CH_3)_2-OH$ | H | H | $CH_3$ | $-OCHF_2$ | CH | O | |
| 5.40 | $-C(CH_3)_2-OH$ | H | H | $OCH_3$ | $-OCH_2-CF_3$ | N | O | |
| 5.41 | $C_2H_5$ | H | H | $OCH_3$ | $OCH_3$ | CH | O | |
| 5.42 | $C_2H_5$ | H | H | $CH_3$ | $CH_3$ | CH | O | |
| 5.43 | $C_2H_5$ | H | H | $CH_3$ | $OCH_3$ | CH | O | |
| 5.44 | $C_2H_5$ | H | H | $OCH_3$ | $OCH_3$ | N | O | |
| 5.45 | $C_2H_5$ | H | H | $CH_3$ | $OCH_3$ | N | O | |
| 5.46 | $C_2H_5$ | H | H | $OCH_3$ | $-N(CH_3)_2$ | N | O | |
| 5.47 | $C_2H_5$ | H | H | $CH_3$ | $-OCHF_2$ | CH | O | |
| 5.48 | $C_2H_5$ | H | H | $OCH_3$ | $-OCH_2-CF_3$ | N | O | |
| 5.49 | 2-Thienyl | H | H | $OCH_3$ | $OCH_3$ | CH | O | |
| 5.50 | 2-Thienyl | H | H | $CH_3$ | $CH_3$ | CH | O | |
| 5.51 | 2-Thienyl | H | H | $CH_3$ | $OCH_3$ | CH | O | |
| 5.52 | 2-Thienyl | H | H | $OCH_3$ | $OCH_3$ | N | O | |
| 5.53 | 2-Thienyl | H | H | $CH_3$ | $OCH_3$ | N | O | |
| 5.54 | 2-Thienyl | H | H | $OCH_3$ | $-N(CH_3)_2$ | N | O | |
| 5.55 | 2-Thienyl | H | H | $CH_3$ | $-OCHF_2$ | CH | O | |
| 5.56 | 2-Thienyl | H | H | $OCH_3$ | $-OCH_2-CF_3$ | N | O | |

Tabelle 5 (Fortsetzung)

| Nr. | R | $R_2$ | $R_1$ | $R_3$ | $R_4$ | E | Z | Smp. [°C] |
|---|---|---|---|---|---|---|---|---|
| 5.57 | 2-Pyridyl | H | H | $OCH_3$ | $OCH_3$ | CH | O | |
| 5.58 | 2-Pyridyl | H | H | $CH_3$ | $CH_3$ | CH | O | |
| 5.59 | 2-Pyridyl | H | H | $CH_3$ | $OCH_3$ | CH | O | |
| 5.60 | 2-Pyridyl | H | H | $OCH_3$ | $OCH_3$ | N | O | |
| 5.61 | 2-Pyridyl | H | H | $CH_3$ | $OCH_3$ | N | O | |
| 5.62 | 2-Pyridyl | H | H | $OCH_3$ | $-N(CH_3)_2$ | N | O | |
| 5.63 | 2-Pyridyl | H | H | $CH_3$ | $-OCHF_2$ | CH | O | |
| 5.64 | 2-Pyridyl | H | H | $OCH_3$ | $-OCH_2-CF_3$ | N | O | |
| 5.65 | $CH_3$ | H | H | Cl | $OCH_3$ | CH | O | |
| 5.66 | $CH_3$ | H | H | $OCH_3$ | $-OCHF_2$ | CH | O | |
| 5.67 | $CH_3$ | H | H | Cl | $-OCHF_2$ | CH | O | |
| 5.68 | $CH_3$ | H | H | $-OCHF_2$ | $-OCHF_2$ | CH | O | |
| 5.69 | $CH_3$ | H | H | $OCH_3$ | $-N(CH_3)_2$ | CH | O | |
| 5.70 | $CH_3$ | H | H | $OCH_3$ | $CF_3$ | CH | O | |
| 5.71 | $CH_3$ | H | H | $OCH_3$ | $CH_2F$ | CH | O | |
| 5.72 | $CH_3$ | H | H | $OCH_3$ | $SCH_3$ | CH | O | |
| 5.73 | $CH_3$ | H | H | $OCH_3$ | $-SCHF_2$ | CH | O | |
| 5.74 | $CH_3$ | H | H | $-OCHF_2$ | $-N(CH_3)_2$ | CH | O | |
| 5.75 | $CH_3$ | H | H | $OCH_3$ | $-NHCH_3$ | N | O | |
| 5.76 | $CH_2$ | H | H | $OCH_3$ | $C_2H_5$ | N | O | |
| 5.77 | $CH_3$ | H | H | $OCH_3$ | $-OCH_2-CH_2Cl$ | N | O | |
| 5.78 | $CH_3$ | H | H | $CH_3$ | $-O(CH_2)_2-N(CH_3)_2$ | N | O | |
| 5.79 | $CH_3$ | H | H | $CH_3$ | $-O(CH_2)_2-N(C_2H_5)_2$ | N | O | |
| 5.80 | $CH_3$ | H | H | $OCH_3$ | $-O(CH_2)_2-N(CH_3)_2$ | N | O | |
| 5.81 | $CH_3$ | H | H | $OCH_3$ | $-O(CH_2)_2-N(C_2H_5)_2$ | N | O | |
| 5.82 | $CH_3$ | H | H | $CH_3$ | $-O(CH_2)_2-N(CH_3)_2$ | CH | O | |
| 5.83 | $CH_3$ | H | H | $CH_3$ | $-O(CH_2)_2-N(C_2H_5)_2$ | CH | O | |
| 5.84 | $CH_3$ | H | H | $OCH_3$ | $-O(CH_2)_2-N(CH_3)_2$ | CH | O | |
| 5.85 | $CH_3$ | H | H | $OCH_3$ | $-O(CH_2)_2-N(C_2H_5)_2$ | CH | O | |
| 5.86 | H | H | H | Cl | $OCH_3$ | CH | O | |

Tabelle 5 (Fortsetzung)

| Nr. | R | $R_2$ | $R_1$ | $R_3$ | $R_4$ | E | Z | Smp.[°C] |
|---|---|---|---|---|---|---|---|---|
| 5.87 | H | H | H | $OCH_3$ | $-OCHF_2$ | CH | O | |
| 5.88 | H | H | H | Cl | $-OCHF_2$ | CH | O | |
| 5.89 | H | H | H | $-OCHF_2$ | $-OCHF_2$ | CH | O | |
| 5.90 | H | H | H | $OCH_3$ | $-N(CH_3)_2$ | CH | O | |
| 5.91 | H | H | H | $OCH_3$ | $CF_3$ | CH | O | |
| 5.92 | H | H | H | $OCH_3$ | $CH_2F$ | CH | O | |
| 5.93 | H | H | H | $OCH_3$ | $SCH_3$ | CH | O | |
| 5.94 | H | H | H | $OCH_3$ | $-SCHF_2$ | CH | O | |
| 5.95 | H | H | H | $-OCHF_2$ | $-N(CH_3)_2$ | CH | O | |
| 5.96 | H | H | H | $OCH_3$ | $-NHCH_3$ | N | O | |
| 5.97 | H | H | H | $OCH_3$ | $C_2H_5$ | N | O | |
| 5.98 | H | H | H | $OCH_3$ | $-OCH_2-CH_2Cl$ | N | O | |
| 5.99 | H | H | H | $CH_3$ | $OCH_3$ | N | S | |
| 5.100 | H | H | H | $CH_3$ | $OCH_3$ | CH | S | |
| 5.101 | H | H | H | $CH_3$ | $CH_3$ | CH | S | |
| 5.102 | H | H | H | $OCH_3$ | $-N(CH_3)_2$ | N | S | |
| 5.103 | H | H | H | $OCH_3$ | $OCH_3$ | N | S | |
| 5.104 | $CH_3$ | 5-F | H | $OCH_3$ | $OCH_3$ | CH | O | |
| 5.105 | $CH_3$ | 5-F | H | $CH_3$ | $CH_3$ | CH | O | |
| 5.106 | $CH_3$ | 5-F | H | $CH_3$ | $-OCHF_2$ | CH | O | |
| 5.107 | $CH_3$ | 5-F | H | $CH_3$ | $-OCH_3$ | N | O | |
| 5.108 | $CH_3$ | 5-F | H | $OCH_3$ | $-N(CH_3)_2$ | N | O | |
| 5.109 | H | 5-F | H | $OCH_3$ | $OCH_3$ | CH | O | |
| 5.110 | H | 5-F | H | $CH_3$ | $CH_3$ | CH | O | |
| 5.111 | H | 5-F | H | $CH_3$ | $-OCHF_2$ | CH | O | |
| 5.112 | H | 5-F | H | $CH_3$ | $OCH_3$ | N | O | |
| 5.113 | H | 5-F | H | $OCH_3$ | $-N(CH_3)_2$ | N | O | |
| 5.114 | H | 5-F | H | $OCH_3$ | $CH_3$ | CH | O | |
| 5.115 | $-C(CH_3)_2-OH$ | 5-F | H | $OCH_3$ | $OCH_3$ | CH | O | |
| 5.116 | $-C(CH_3)_2-OH$ | 5-F | H | $OCH_3$ | CH | CH | O | |

Tabelle 5 (Fortsetzung)

| Nr. | R | $R_2$ | $R_1$ | $R_3$ | $R_4$ | E | Z | Smp. [°C] |
|---|---|---|---|---|---|---|---|---|
| 5.117 | $-C(CH_3)_2-OH$ | 5-F | H | $CH_3$ | $CH_3$ | CH | O | |
| 5.118 | $-C(CH_3)_2-OH$ | 5-F | H | $CH_3$ | $-OCHF_2$ | CH | O | |
| 5.119 | $-C(CH_3)_2-OH$ | 5-F | H | $CH_3$ | $-OCH_3$ | N | O | |
| 5.120 | $-C(CH_3)_3-OH$ | 5-F | H | $OCH_3$ | $-N(CH_3)_2$ | N | O | |
| 5.121 | $-C(CH_3)_2-OH$ | 5-F | H | $OCH_3$ | $OCH_3$ | N | O | |
| 5.122 | $C_6H_5$ | 5-F | H | $OCH_3$ | $OCH_3$ | CH | O | |
| 5.123 | $C_6H_5$ | 5-F | H | $CH_3$ | $OCH_3$ | N | O | |
| 5.124 | $C_6H_6$ | 5-F | H | $OCH_3$ | $OCH_3$ | N | O | |
| 5.125 | $C_6H_5$ | 5-F | H | $OCH_3$ | $-N(CH_3)_2$ | N | O | |
| 5.126 | 2-Thienyl | 5-F | H | $OCH_3$ | $OCH_3$ | CH | O | |
| 5.127 | 2-Thienyl | 5-F | H | $OCH_3$ | $CH_3$ | N | O | |
| 5.128 | 2-Furyl | 5-F | H | $OCH_3$ | $OCH_3$ | CH | O | |
| 5.129 | 2-Furyl | 5-F | H | $CH_3$ | $OCH_3$ | N | O | |
| 5.130 | $CH_3$ | 6-Cl | H | $OCH_3$ | $OCH_3$ | CH | O | |
| 5.131 | $CH_3$ | 6-Cl | H | $CH_3$ | $CH_3$ | CH | O | |
| 5.132 | $CH_3$ | 6-Cl | H | $CH_3$ | $OCH_3$ | CH | O | |
| 5.133 | $CH_3$ | 6-Cl | H | $CH_3$ | $OCHF_2$ | CH | O | |
| 5.134 | $CH_3$ | 6-Cl | H | $OCH_3$ | $OCH_3$ | N | O | |
| 5.135 | $CH_3$ | 6-Cl | H | $CH_3$ | $OCH_3$ | N | O | |
| 5.136 | $CH_3$ | $6-COOCH_3$ | H | $CH_3$ | $OCH_3$ | N | O | |
| 5.137 | $CH_3$ | $6-COOCH_3$ | H | $CH_3$ | $OCH_3$ | CH | O | |
| 5.138 | $CH_3$ | $6-OCH_3$ | H | $CH_3$ | $OCH_3$ | N | O | |
| 5.139 | $CH_3$ | $6-OCH_3$ | H | $CH_3$ | $OCH_3$ | CH | O | |
| 5.140 | $CH_3$ | $6-OCHF_2$ | H | $CH_3$ | $OCH_3$ | N | O | |
| 5.141 | $CH_3$ | $6-OCHF_2$ | H | $CH_3$ | $OCH_3$ | CH | O | |
| 5.142 | $CH_3$ | $6-CF_3$ | H | $CH_3$ | $OCH_3$ | N | O | |
| 5.143 | $CH_3$ | $6-CF_3$ | H | $CH_3$ | $OCH_3$ | CH | O | |
| 5.144 | H | 6-Cl | H | $CH_3$ | $OCH_3$ | N | O | |

Tabelle 6

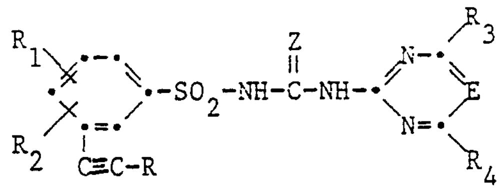

| Nr. | R | $R_2$ | $R_1$ | $R_3$ | $R_4$ | E | Z | Smp. [°C] |
|---|---|---|---|---|---|---|---|---|
| 6.1 | $CH_3$ | 6-Cl | H | $CH_3$ | $-OCHF_2$ | CH | O | |
| 6.2 | $CH_3$ | 6-Cl | H | $CH_3$ | $OCH_3$ | N | O | |
| 6.3 | $CH_3$ | 6-Cl | H | $OCH_3$ | $OCH_3$ | CH | O | |
| 6.4 | H | 6-Cl | H | $CH_3$ | $-OCHF_2$ | CH | O | |
| 6.5 | H | 6-Cl | H | $CH_3$ | $OCH_3$ | N | O | |
| 6.6 | H | 6-Cl | H | $OCH_3$ | $OCH_3$ | CH | O | |
| 6.7 | $-C(CH_3)_2-OH$ | 6-Cl | H | $OCH_3$ | $OCH_3$ | CH | O | |
| 6.8 | $C_6H_5$ | 6-Cl | H | $OCH_3$ | $OCH_3$ | CH | O | |
| 6.9 | $CH_3$ | 6-F | H | $OCH_3$ | $OCH_3$ | CH | O | |
| 6.10 | $CH_3$ | 6-F | H | $CH_3$ | $CH_3$ | CH | O | |
| 6.11 | $CH_3$ | 6-F | H | $CH_3$ | $-OCHF_2$ | CH | O | |
| 6.12 | $CH_3$ | 6-F | H | $CH_3$ | $CH_3$ | N | O | |
| 6.13 | $CH_3$ | 6-F | H | $OCH_3$ | $-N(CH_3)_2$ | N | O | |
| 6.14 | H | 6-F | H | $OCH_3$ | $OCH_3$ | CH | O | |
| 6.15 | H | 6-F | H | $CH_3$ | $CH_3$ | CH | O | |
| 6.16 | H | 6-F | H | $CH_3$ | $-OCHF_2$ | CH | O | |
| 6.17 | H | 6-F | H | $CH_3$ | $OCH_3$ | N | O | |
| 6.18 | H | 6-F | H | $OCH_3$ | $-N(CH_3)_2$ | N | O | |
| 6.19 | H | 6-F | H | $OCH_3$ | $CH_3$ | CH | O | |
| 6.20 | $-C(CH_3)_2-OH$ | 6-F | H | $OCH_3$ | $OCH_3$ | CH | O | |
| 6.21 | $-C(CH_3)_2-OH$ | 6-F | H | $OCH_3$ | $CH_3$ | CH | O | |
| 6.22 | $-C(CH_3)_2-OH$ | 6-F | H | $CH_3$ | $CH_3$ | CH | O | |
| 6.23 | $-C(CH_3)_2-OH$ | 6-F | H | $CH_3$ | $-OCHF_2$ | CH | O | |
| 6.24 | $-C(CH_3)_2-OH$ | 6-F | H | $CH_3$ | $OCH_3$ | N | O | |
| 6.25 | $-C(CH_3)_2-OH$ | 6-F | H | $OCH_3$ | $-N(CH_3)_2$ | N | O | |
| 6.26 | $-C(CH_3)_2-OH$ | 6-F | H | $OCH_3$ | $OCH_3$ | N | O | |

Tabelle 6 (Fortsetzung)

| Nr. | R | $R_2$ | $R_1$ | $R_3$ | $R_4$ | E | Z | Smp. [°C] |
|---|---|---|---|---|---|---|---|---|
| 6.27 | $C_6H_5$ | 6-F | H | $OCH_3$ | $OCH_3$ | CH | O | |
| 6.28 | $C_6H_5$ | 6-F | H | $CH_3$ | $OCH_3$ | N | O | |
| 6.29 | $C_6H_5$ | 6-F | H | $OCH_3$ | $OCH_3$ | N | O | |
| 6.30 | $C_6H_5$ | 6-F | H | $OCH_3$ | $-N(CH_3)_2$ | N | O | |
| 6.31 | 2-Thienyl | 6-F | H | $OCH_3$ | $OCH_3$ | CH | O | |
| 6.32 | 2-Thienyl | 6-F | H | $CH_3$ | $OCH_3$ | N | O | |
| 6.33 | 2-Furyl | 6-F | H | $OCH_3$ | $OCH_3$ | CH | O | |
| 6.34 | 2-Furyl | 6-F | H | $CH_3$ | $OCH_3$ | N | O | |
| 6.35 | $CH_3$ | H | H | $CH_3$ | $OCH_3$ | N | O | |
| 6.36 | $CH_3$ | H | H | $CH_3$ | $OCH_3$ | CH | O | |
| 6.37 | H | H | H | $CH_3$ | $OCH_3$ | CH | O | |
| 6.38 | $CH_3$ | $6-COOCH_3$ | H | $CH_3$ | $OCH_3$ | CH | O | |
| 6.39 | $CH_3$ | $6-COOCH_3$ | H | $CH_3$ | $OCH_3$ | N | O | |
| 6.40 | $CH_3$ | $6-CF_3$ | H | $CH_3$ | $OCH_3$ | N | O | |
| 6.41 | $CH_3$ | $6-CF_3$ | H | $CH_3$ | $OCH_3$ | CH | O | |
| 6.42 | $CH_3$ | $6-OCH_3$ | H | $CH_3$ | $OCH_3$ | N | O | |
| 6.43 | $CH_3$ | $6-OCH_3$ | H | $CH_3$ | $OCH_3$ | CH | O | |
| 6.44 | $CH_3$ | $6-OCHF_2$ | H | $CH_3$ | $OCH_3$ | N | O | |
| 6.45 | $CH_3$ | $6-OCHF_2$ | H | $CH_3$ | $OCH_3$ | CH | O | |
| 6.46 | $CH_3$ | $6-CH_3$ | H | $CH_3$ | $OCH_3$ | N | O | |
| 6.47 | $CH_3$ | $6-CH_3$ | H | $CH_3$ | $OCH_3$ | CH | O | |

## Formulierungsbeispiele

Beispiel 2: Formulierungsbeispiele für Wirkstoffe der Formel I (% = Gewichtsprozent)

a) Spritzpulver

| | a) | b) | c) |
|---|---|---|---|
| Wirkstoff | 20 % | 60 % | 0,5 % |
| Na-Ligninsulfonat | 5 % | 5 % | 5 % |
| Na-Laurylsulfat | 3 % | - | - |
| Na-Diisobutylnaphthalinsulfonat | - | 6 % | 6 % |
| Octylphenolpolyäthylenglykoläther (7-8 Mol AeO) | - | 2 % | 2 % |
| Hochdisperse Kieselsäure | 5 % | 27 % | 27 % |
| Kaolin | 67 % | - | - |
| Natriumchlorid | - | - | 59,5 % |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| b) Emulsion-Konzentrat | a) | b) |
|---|---|---|
| Wirkstoff | 10 % | 1 % |
| Octylphenolpolyäthylenglykoläther (4-5 Mol AeO) | 3 % | 3 % |
| Ca-Dodecylbenzolsulfonat | 3 % | 3 % |
| Ricinusölpolyglykoläther (36 Mol AeO) | 4 % | 4 % |
| Cyclohexanon | 30 % | 10 % |
| Xylolgemisch | 50 % | 79 % |

Aus diesem Konzentrat können durch Verdünnen mit Wasser Emulsionen jeder gewünschten Konzentration hergestellt werden.

| c) Stäubemittel | a) | b) |
|---|---|---|
| Wirkstoff | 0,1 % | 1 % |
| Talkum | 99,9 % | - |
| Kaolin | - | 99 % |

Man erhält anwendungsfertige Stäubemittel, indem der Wirkstoff mit dem Träger vermischt und auf einer geeigneten Mühle vermahlen wird.

| d) Extruder Granulat | a) | b) |
|---|---|---|
| Wirkstoff | 10 % | 1 % |
| Na-Ligninsulfonat | 2 % | 2 % |
| Carboxymethylcellulose | 1 % | 1 % |
| Kaolin | 87 % | 96 % |

Der Wirkstoff wird mit den Zusatzstoffen vermischt, vermahlen und mit Wasser angefeuchtet. Dieses Gemisch wird extrudiert und anschliessend im Luftstrom getrocknet.

| e) Umhüllungs-Granulat | |
|---|---|
| Wirkstoff | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| f) Suspensions-Konzentrat | a) | b) |
|---|---|---|
| Wirkstoff | 40 % | 5 % |
| Aethylenglykol | 10 % | 10 % |
| Nonylphenolpolyäthylenglykoläther (15 Mol AeO) | 6 % | 1 % |

(Fortsetzung)

| | | |
|---|---|---|
| Na-Ligninsulfonat | 10 % | 5 % |
| Carboxymethylcellulose | 1 % | 1 % |
| 37%ige wässrige Formaldehyd-Lösung | 0,2 % | 0,2 % |
| Silikonöl in Form einer 75%igen wässrigen Emulsion | 0,8 % | 0,8 % |
| Wasser | 32 % | 77 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

g) Salzlösung

| | |
|---|---|
| Wirkstoff | 5 % |
| Isopropylamin | 1 % |
| Octylphenolpolyäthylenglykoläther (78 Mol AeO) | 3 % |
| Wasser | 91 % |

## Biologische Beispiele

### Beispiel 3 : Nachweis der Herbizidwirkung vor dem Auflaufen der Pflanzen

Kunststofftöpfe werden mit expandiertem Vermiculit (Dichte : 0,135 g/cm$^3$, Wasserabsorptionsvermögen : 0,565 1/1) gefüllt. Nach dem Sättigen des nicht adsorptiven Vermiculits mit einer wässrigen Wirkstoffemulsion in deionisiertem Wasser, die die Wirkstoffe in einer Konzentration von 70,8 ppm enthält, werden Samen der folgenden Pflanzen auf die Oberfläche gesät : Nasturtium officinalis, Agrostis tenuis, Stellaria media und Digitaria sanguinalis. Die Versuchsgefässe werden anschliessend in einer Klimakammer bei 20 °C, einer Beleuchtung von ca. 20 kLux und einer relativen Luftfeuchtigkeit von 70 % gehalten. Während der Keimphase von 4 bis 5 Tagen werden die Töpfe zur Erhöhung der örtlichen Luftfeuchtigkeit mit lichtdurchlässigem Material abgedeckt und mit deionisiertem Wasser gegossen. Nach dem 5. Tag wird dem Gieswasser 0,5 % eines handelsüblichen Flüssigdüngers (®Greenzit) zugesetzt. 12 Tage nach der Aussaat wird der Versuch ausgewertet und die Wirkung auf die Versuchspflanzen nach dem folgenden Massstab bewertet :

1 : Pflanze nicht gekeimt oder total abgestorben
2-3 : sehr starke Wirkung
4-6 : mittlere Wirkung
7-8 : schwache Wirkung
9 : keine Wirkung (wie unbehandelte Kontrolle).

pre-emergente Wirkung

Konzentration der Wirkstoffemulsion : 70,8 ppm

| Testpflanze / Wirkstoff Nr. | Nasturtium | Stellaria | Agrostis | Digitaria |
|---|---|---|---|---|
| 5.1 | 1 | 1 | 1 | 1 |
| 5.2 | 1 | 2 | 1 | 2 |
| 5.3 | 2 | 2 | 1 | 3 |
| 5.5 | 2 | 3 | 2 | 4 |
| 5.17 | 2 | 2 | 2 | 3 |
| 5.33 | 1 | 2 | 1 | 2 |

Beispiel 4 : Nachweis der Herbizidwirkung nach dem Auflaufen der Pflanzen (Kontaktwirkung)

Eine Anzahl Unkräuter und Kulturpflanzen, sowohl monokotyle wie dikotyle, wurden nach dem Auflaufen im 4- bis 6-Blattstadium mit einer wässrigen Wirkstoffdispersion in Dosierungen von 4 kg AS/ha gespritzt und dann bei 24° bis 26 °C und 45-60 % relativer Lufteuchtigkeit gehalten. 15 Tage nach der Behandlung wird der Versuch ausgewertet und nach dem gleichen Massstab wie im pre-emergenten Versuch bewertet.

post-emergente Wirkung

| Verb. Nr. | Avena | Solanum | Sinapis | Stellaria | Phaseolus |
|---|---|---|---|---|---|
| 5.1 | 6 | 2 | 2 | 4 | 2 |
| 5.33 | 6 | 2 | 3 | 5 | 3 |

Beispiel 5 : Nachweis der Keimhemmung an Lagerkartoffeln

Eine Anzahl im Handel erhältliche Kartoffeln der Sorte « Urgenta » ohne Keime werden gewaschen und abgetrocknet. Danach werden die Kartoffeln für jeweils eine Minute in Wirkstoffemulsionen verschiedener Konzentration getaucht, in Kunststoffschalen auf Filterpapier ausgelegt und bei Temperaturen von 14° und 21 °C im Dunkeln bei 50 % relativer Luftfeuchtigkeit gehalten. Die Auswertung erfolgte 34 Tage nach der Applikation. Gleichzeitig wird der Gewichtsverlust der Knollen und das Gewicht der Keime im Vergleich zur unbehandelten Kontrolle ermittelt. Die erfindungsgemässen Verbindungen zeigten in diesem Versuch eine vollständige Verhinderung der Keimbildung. Gleichzeitig betrug der Gewichtsverlust der Kartoffeln weniger als 10 % des Gewichtsverlustes der Kontrollkartoffeln.

Beispiel 6 : Nachweis der Wuchshemmung bei tropischen Bodendeckern-Leguminosen (cover crops)

Die Versuchspflanzen (centrosema plumieri und centrosoma pubescens) werden bis zum ausgewachsenen Stadium herangezogen und bis auf eine Höhe von 60 cm zurückgeschnitten. Nach 7 Tagen wird der Wirkstoff als wässrige Emulsion gespritzt. Die Versuchspflanzen werden bei 70 % relativer Luftfeuchtigkeit und 6 000 lux Kunstlicht, pro Tag 14 Stunden, bei Temperaturen von 27° bei Tag und 21 °C bei Nacht gehalten. 4 Wochen nach der Applikation wird der Versuch ausgewertet. Es werden dabei der Neuzuwachs im Vergleich zur Kontrolle abgeschätzt und gewogen und die Phytotoxizität bewertet. In diesem Versuch zeigen die mit den Wirkstoffen der Formel I behandelten Pflanzen eine deutliche Reduktion des Neuzuwachses (weniger als 20 % des Neuzuwachses bei unbehandelten Kontrollpflanzen), ohne dass dabei die Versuchspflanzen geschädigt wurden.

**Patentansprüche** (für die Vertragsstaaten : BE, CH, DE, FR, GB, IT, LI, NL)

1. N-Phenylsulfonyl-N'-pyrimidinyl- und -triazinylharnstoffe der allgemeinen Formel I

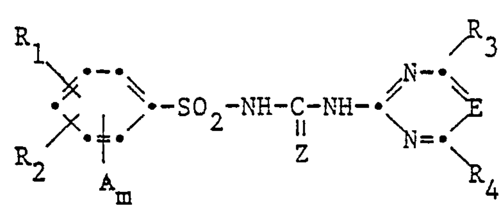

(I)

worin

A einen Rest der Formel —C≡C—R,

m die Zahl eins oder zwei,

E die Methingruppe oder Stickstoff,

Z Sauerstoff oder Schwefel,

R Wasserstoff ; gegebenenfalls durch Halogen, Hydroxyl, $C_1$-$C_4$-Alkoxy, $C_1$-$C_4$-Alkylthio, $C_1$-$C_4$-Halogenalkoxy, $C_3$-$C_9$-Cycloalkyl, Cyan, —$COOR_6$, —$CONR_7R_8$ oder seinerseits gegebenenfalls durch Halogen, $C_1$-$C_4$-Alkyl, $C_1$-$C_4$-Alkoxy, Nitro, Cyan oder Trifluormethyl substituiertes Phenyl, substituiertes verzweigtes oder unverzweigtes $C_1$-$C_9$-Alkyl ; gegebenenfalls durch Halogen, Hydroxyl, $C_1$-$C_4$-Alkyl, $C_1$-

$C_4$-Alkoxy, $C_1$-$C_4$-Alkylthio, Cyan, —$COOR_6$, —$CONR_7R_8$ substituiertes $C_3$-$C_9$-Cycloalkyl ; gegebenenfalls durch Halogen, Nitro, Cyan, $C_1$-$C_4$-Alkyl, $C_1$-$C_4$-Alkoxy, $C_1$-$C_4$-Alkylthio, Trifluormethyl, —$COOR_6$ oder —$CONR_7R_8$ substituiertes Phenyl ; oder einen gegebenenfalls durch Halogen, $C_1$-$C_4$-Alkyl, $C_1$-$C_4$-Alkoxy, —$COOR_6$ oder —$CONR_7R_8$ substituierten 5- bis 6-gliedrigen aromatischen Heterocyclus,

$R_1$ Wasserstoff, Halogen, $C_1$-$C_5$-Alkyl, $C_2$-$C_5$-Alkenyl oder einen Rest —Y—$R_5$,

$R_2$ Wasserstoff, Halogen, $C_1$-$C_5$-Alkyl, $C_2$-$C_5$-Alkenyl, $C_1$-$C_4$-Halogenalkyl, oder einen Rest —Y—$R_5$, —$COOR_6$, —$NO_2$ oder —CO—$NR_7$—$R_8$,

$R_3$ und $R_4$ unabhängig voneinander Wasserstoff, $C_1$-$C_4$-Alkyl, $C_1$-$C_4$-Alkoxy, $C_1$-$C_4$-Alkylthio, $C_1$-$C_4$-Halogenalkyl, $C_1$-$C_4$-Halogenalkoxy, $C_1$-$C_4$-Halogenalkylthio, Halogen, $C_2$-$C_5$-Alkoxyalkyl, —$NR_9R_{10}$ oder —O—$CH_2$—$CH_2$—$NR_9R_{10}$,

$R_5$ und $R_6$ unabhängig voneinander $C_1$-$C_5$-Alkyl, $C_2$-$C_5$-Alkenyl oder $C_2$-$C_6$-Alkinyl, $C_1$-$C_5$-Halogenalkyl, $C_2$-$C_5$-Halogenalkenyl oder $C_2$-$C_6$-Alkoxyalkyl,

$R_7$ und $R_8$ unabhängig voneinander Wasserstoff, $C_1$-$C_5$-Alkyl, $C_2$-$C_5$-Alkenyl oder $C_2$-$C_6$-Alkinyl,

$R_9$ Wasserstoff, Methyl oder Aethyl,

$R_{10}$ Wasserstoff, $C_1$-$C_4$-Alkyl, $C_1$-$C_4$-Alkoxy, Methoxymethyl, Cyanomethyl, Cyanoäthyl, $C_3$-$C_5$-Alkenyl oder $C_3$-$C_6$-Alkinyl und

Y Sauerstoff, Schwefel, eine Sulfinyl- oder Sulfonylbrücke bedeuten,

sowie die Salze dieser Verbindungen.

2. Verbindungen gemäss Anspruch 1, dadurch gekennzeichnet, dass Z Sauerstoff und m die Zahl eins bedeutet, und dass die Reste $R_3$ und $R_4$ zusammen nicht mehr als 4 Kohlenstoffatome enthalten.

3. Verbindungen gemäss Anspruch 2, dadurch gekennzeichnet, dass nur ein Rest A in 2- oder 3-Stellung zum Sulfonylrest vorhanden ist, die Reste $R_3$ und $R_4$ zusammen nicht mehr als 4 Kohlenstoffatome enthalten, $R_1$ Wasserstoff bedeutet und der Rest $R_2$ in der 5- oder 6-Stellung zur Sulfonylgruppe steht.

4. Verbindungen gemäss Anspruch 3, dadurch gekennzeichnet, dass $R_2$ Wasserstoff, Chlor, Fluor, Methyl, Nitro, Trifluormethyl, $C_1$-$C_4$-Halogenalkoxy, $C_1$-$C_4$-Alkoxycarbonyl oder $C_1$-$C_4$-Alkoxy und $R_3$ und $R_4$ jeweils Wasserstoff, $C_1$-$C_4$-Alkyl, $C_1$-$C_4$-Alkoxy, $C_1$-$C_4$-Alkylthio, Dialkylamino, $C_1$-$C_3$-Halogenalkoxy, Halogen oder Alkoxyalkyl bedeuten, wobei $R_3$ und $R_4$ zusammen höchstens 4 Kohlenstoffatome enthalten und R Wasserstoff oder gegebenenfalls durch Halogen, Hydroxyl, Methoxy, Methylthio, $C_1$-$C_4$-Halogenalkoxy, Cyclopropyl, Cyan, Methoxycarbonyl substituiertes verzweigtes oder unerzweigtes $C_1$-$C_4$-Alkyl oder Phenyl, Pyridyl, Thienyl oder Furyl bedeutet, der Rest R in 2- oder 3-Stellung und der Rest $R_2$ in 6-Stellung zur Sulfonylgruppe steht.

5. N-[2-(1-Propinyl)-phenylsulfonyl]-N'-(4,6-dimethoxy-pyrimidin-2-yl)-harnstoff gemäss Anspruch 1.

6. N-[2-(1-Propinyl)-6-chlor-phenylsulfonyl]-N'-(4-methoxy-6-methyltriazin-2-yl)-harnstoff gemäss Anspruch 1.

7. N-[2-(1-Propionyl)-phenylsulfonyl]-N'-(4-methoxy-6-methyl-pyrimidin-2-yl)-harnstoff gemäss Anspruch 1.

8. Verfahren zur Herstellung der Verbindung der Formel I, Anspruch 1, dadurch gekennzeichnet, dass man entweder

a) ein Phenylsulfonamid der Formel II

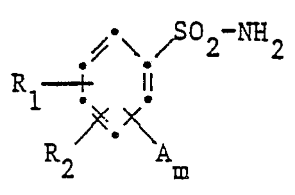

(II)

worin A, $R_1$, $R_2$ und m die unter Formel I gegebene Bedeutung haben, in Gegenwart einer Base mit einem N-Pyrimidinyl- oder -Triazinylcarbamat der Formel

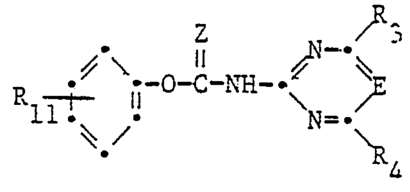

(III)

worin E, $R_3$, $R_4$ und Z die unter Formel I gegebene Bedeutung haben und $R_{11}$ für Wasserstoff, Halogen, $C_1$-$C_4$-Alkyl, $C_1$-$C_4$-Alkoxy, Nitro, Cyan oder Trifluormethyl steht, oder

b) ein Phenylsulfonylisocyanat oder -isothiocyanat der Formel IV

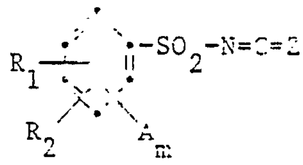

(IV)

worin A, $R_1$, $R_2$, m und Z die unter Formel I gegebene Bedeutung haben, gegebenenfalls in Gegenwart einer Base, mit einem Amin der Formel V

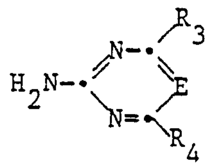

(V)

worin R, $R_3$ und $R_4$ die unter Formel I gegebene Bedeutung haben, oder

c) Sulfonamide der oben angegebenen Formel II gegebenenfalls in Gegenwart einer Base mit einem Isocyanat oder Isothiocyanat der Formel VI

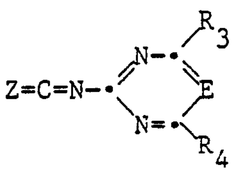

(VI)

worin E, $R_3$, $R_4$ und Z die unter Formel I gegebenen Bedeutungen haben, oder

d) ein N-Phenylsulfonylcarbamat der Formel VII

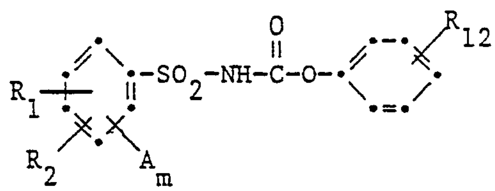

(VII)

worin A, $R_1$, $R_2$ und m die unter Formel I gegebene Bedeutung haben und $R_{12}$ für Wasserstoff, Halogen, $C_1$-$C_4$-Alkyl, $C_1$-$C_4$-Alkoxy, Nitro, Cyan oder Trifluormethyl steht, mit einem Amin der oben angegebenen Formel V umsetzt und gegebenenfalls in ihre Salze überführt, indem man einen Sulfonylharnstoff der Formel I mit einem Amin, einem Alkalimetall- oder Erdalkalimetallhydroxid oder einer quaternären Ammoniumbase umsetzt.

9. Phenylsulfonamide der Formel II

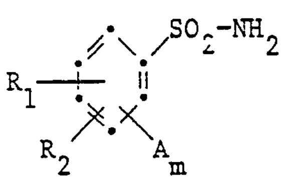

(II)

worin A, $R_1$, $R_2$ und m die unter Formel I, Anspruch 1, gegebene Bedeutung haben.

10. Ein herbizides und den Pflanzenwuchs hemmendes Mittel, dadurch gekennzeichnet, dass es neben Träger- und/oder anderen Zuschlagstoffen als Wirkstoff mindestens einen N-Phenylsulfonyl-N'-triazinyl- oder -pyrimidinyl-harnstoff der Formel I, Anspruch 1, enthält.

11. Die Verwendung der N-Phenylsulfonyl-N'-triazinyl- oder -pyrimidinyl-harnstoffe der Formel I, Anspruch 1, oder sie enthaltender Mittel zur Bekämpfung unerwünschten Pflanzenwachstums.

12. Die Verwendung der N-Phenylsulfonyl-N'-triazinyl- oder pyrimidinyl-harnstoffe der Formel I, Anspruch 1, oder sie enthaltender Mittel zur Hemmung des Pflanzenwachstums.

13. Die Verwendung der N-Phenylsulfonyl-N'-triazinyl- oder pyrimidinyl-harnstoffe der Formel I, oder sie enthaltender Mittel, gemäss Anspruch 11, zur selektiven pre- oder post-emergenten Bekämpfung von Unkräutern in Nutzpflanzenkulturen.

14. Die Verwendung der Verbindungen der Formel I oder sie enthaltender Mittel gemäss Anspruch 12 zur Wuchshemmung von Bodendecker-Leguminosen.

15. Die Verwendung der Verbindungen der Formel I oder sie enthaltender Mittel gemäss Anspruch 12 zur Keimhemmung von Lagerkartoffeln.

16. Die Verwendung der Verbindungen der Formel I oder sie enthaltender Mittel gemäss Anspruch 13 in Getreide, Baumwolle, Soja, Mais und Reis.

**Patentansprüche** (für den Vertragsstaat AT)

1. Ein herbizides und den Pflanzenwuchs hemmendes Mittel, dadurch gekennzeichnet, dass es neben Träger- und/oder anderen Zuschlagstoffen als Wirkstoff mindestens einen N-Phenylsulfonyl-N'-triazinyl- oder -pyrimidinyl-harnstoff der Formel I

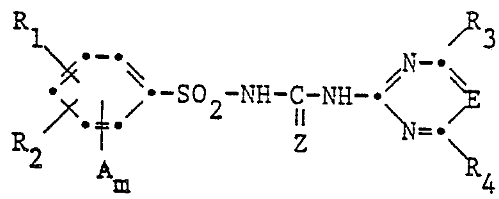

(I)

oder deren Salze enthält, worin

A einen Rest der Formel —C≡C—R,

m die Zahl eins oder zwei,

E die Methingruppe oder Stickstoff,

Z Sauerstoff oder Schwefel,

R Wasserstoff; gegebenenfalls durch Halogen, Hydroxyl, $C_1$-$C_4$-Alkoxy, $C_1$-$C_4$-Alkylthio, $C_1$-$C_4$-Halogenalkoxy, $C_3$-$C_9$-Cycloalkyl, Cyan, $—COOR_6$, $—CONR_7R_8$ oder seinerseits gegebenenfalls durch Halogen, $C_1$-$C_4$-Alkyl, $C_1$-$C_4$-Alkoxy, Nitro, Cyan oder Trifluormethyl substituiertes Phenyl, substituiertes verzweigtes oder unverzweigtes $C_1$-$C_9$-Alkyl; gegebenenfalls durch Halogen, Hydroxyl, $C_1$-$C_4$-Alkyl, $C_1$-$C_4$-Alkoxy, $C_1$-$C_4$-Alkylthio, Cyan, $—COOR_6$, $—CONR_7R_8$ substituiertes $C_3$-$C_9$-Cycloalkyl; gegebenenfalls durch Halogen, Nitro, Cyan, $C_1$-$C_4$-Alkyl, $C_1$-$C_4$-Alkoxy, $C_1$-$C_4$-Alkylthio, Trifluormethyl, $—COOR_6$ oder $—CONR_7R_8$ substituiertes Phenyl; oder einen gegebenenfalls durch Halogen, $C_1$-$C_4$-Alkyl, $C_1$-$C_4$-Alkoxy, $—COOR_6$ oder $—CONR_7R_8$ substituierten 5- bis 6-gliedrigen aromatischen Heterocyclus,

$R_1$ Wasserstoff, Halogen, $C_1$-$C_5$-Alkyl, $C_2$-$C_5$-Alkenyl oder einen Rest $—Y—R_5$,

$R_2$ Wasserstoff, Halogen, $C_1$-$C_5$-Alkyl, $C_2$-$C_5$-Alkenyl, $C_1$-$C_4$-Halogenalkyl, oder einen Rest $—Y—R_5$, $—COOR_6$, $—NO_2$ oder $—CO—NR_7—R_8$,

$R_3$ und $R_4$ unabhängig voneinander Wasserstoff, $C_1$-$C_4$-Alkyl, $C_1$-$C_4$-Alkoxy, $C_1$-$C_4$-Alkylthio, $C_1$-$C_4$-Halogenalkyl, $C_1$-$C_4$-Halogenalkoxy, $C_1$-$C_4$-Halogenalkylthio, Halogen, $C_2$-$C_5$-Alkoxyalkyl, $—NR_9R_{10}$ oder $—O—CH_2—CH_2—NR_9R_{10}$,

$R_5$ und $R_6$ unabhängig voneinander $C_1$-$C_5$-Alkyl, $C_2$-$C_5$-Alkenyl oder $C_2$-$C_6$-Alkinyl, $C_1$-$C_5$-Halogenalkyl, $C_2$-$C_5$-Halogenalkenyl oder $C_2$-$C_6$-Alkoxyalkyl,

$R^7$ und $R^8$ unabhängig voneinander Wasserstoff, $C_1$-$C_5$-Alkyl, $C_2$-$C_5$-Alkenyl oder $C_2$-$C_6$-Alkinyl,

$R_9$ Wasserstoff, Methyl oder Aethyl,

$R_{10}$ Wasserstoff, $C_1$-$C_4$-Alkyl, $C_1$-$C_4$-Alkoxy, Methoxymethyl, Cyanomethyl, Cyanoäthyl, $C_3$-$C_5$-Alkenyl oder $C_3$-$C_6$-Alkinyl und

Y Sauerstoff, Schwefel, eine Sulfinyl- oder Sulfonylbrücke bedeuten.

2. Mittel gemäss Anspruch 1, dadurch gekennzeichnet, dass Z Sauerstoff und m die Zahl eins bedeutet, und dass die Reste $R_3$ und $R_4$ zusammen nicht mehr als 4 Kohlenstoffatome enthalten.

3. Mittel gemäss Anspruch 2, dadurch gekennzeichnet, dass nur ein Rest A in 2- oder 3-Stellung zum Sulfonylrest vorhanden ist, die Reste $R_3$ und $R_4$ zusammen nicht mehr als 4 Kohlenstoffatome enthalten, $R_1$ Wasserstoff bedeutet und der Rest $R_2$ in der 5- oder 6-Stellung zur Sulfonylgruppe steht.

4. Mittel gemäss Anspruch 3, dadurch gekennzeichnet, dass $R_2$ Wasserstoff, Chlor, Fluor, Methyl, Nitro, Trifluormethyl, $C_1$-$C_4$-Halogenalkoxy, $C_1$-$C_4$-Alkoxycarbonyl oder $C_1$-$C_4$-Alkoxy und $R_3$ und $R_4$ jeweils Wasserstoff, $C_1$-$C_4$-Alkyl, $C_1$-$C_4$-Alkoxy, $C_1$-$C_4$-Alkylthio, Dialkylamino, $C_1$-$C_3$-Halogenalkoxy, Halogen oder Alkoxyalkyl bedeuten, wobei $R_3$ und $R_4$ zusammen höchstens 4 Kohlenstoffatome enthalten und R Wasserstoff oder gegebenenfalls durch Halogen, Hydroxyl, Methoxy, Methylthio, $C_1$-$C_4$-Halogenalkoxy, Cyclopropyl, Cyan, Methoxycarbonyl substituiertes verzweigtes oder unverzweigtes $C_1$-$C_4$-Alkyl oder Phenyl, Pyridyl, Thienyl oder Furyl bedeutet, der Rest R in 2- oder 3-Stellung und der Rest $R_2$ in 6-Stellung zur Sulfonylgruppe steht.

5. Mittel gemäss Anspruch 1, dadurch gekennzeichnet, dass es als Wirkstoff N-[2-(1-Propinyl)-phenylsulfonyl]-N'-(4,6-dimethoxy-pyrimidin-2-yl)-harnstoff enthält.

6. Mittel gemäss Anspruch 1, dadurch gekennzeichnet, dass es als Wirkstoff N-[2-(1-Propinyl)-6-chlor-phenylsulfonyl]-N'-(4-methoxy-6-methyl-triazin-2-yl)-harnstoff enthält.

7. Mittel gemäss Anspruch 1, dadurch gekennzeichnet, dass es als Wirkstoff N-[2-(1-Propionyl)-phenylsulfonyl]-N'-(4-methoxy-6-methyl-pyrimidin-2-yl)-harnstoff enthält.

8. Verfahren zur Herstellung der Verbindung der Formel I, gemäss Anspruch 1, dadurch gekennzeichnet, dass man entweder

a) ein Phenylsulfonamid der Formel II

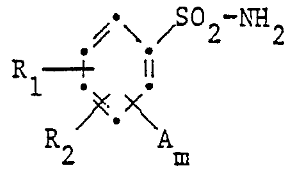

(II)

worin A, $R_1$, $R_2$ und m die unter Formel I gegebene Bedeutung haben, in Gegenwart einer Base mit einem N-Pyrimidinyl- oder -Triazinylcarbamat der Formel

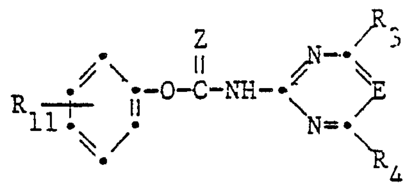

(III)

worin E, $R_3$, $R_4$ und Z die unter Formel I gegebene Bedeutung haben und $R_{11}$ für Wasserstoff, Halogen, $C_1$-$C_4$-Alkyl, $C_1$-$C_4$-Alkoxy, Nitro, Cyan oder Trifluormethyl steht, oder

b) ein Phenylsulfonylisocyanat oder -isothiocyanat der Formel IV

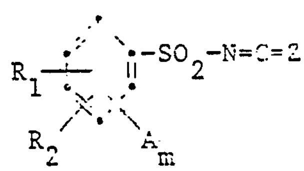

(IV)

worin A, $R_1$, $R_2$, m und Z die unter Formel I gegebene Bedeutung haben, gegebenenfalls in Gegenwart einer Base, mit einem Amin der Formel V

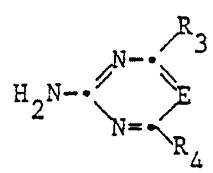

(V)

worin R, $R_3$ und $R_4$ die unter Formel I gegebene Bedeutung haben, oder

c) Sulfonamide der oben angegebenen Formel II gegebenenfalls in Gegenwart einer Base mit einem Isocyanat oder Isothiocyanat der Formel VI

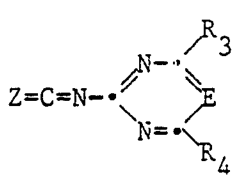

(VI)

worin E, $R_3$, $R_4$ und Z die unter Formel I gegebenen Bedeutungen haben, oder

d) ein N-Phenylsulfonylcarbamat der Formel VII

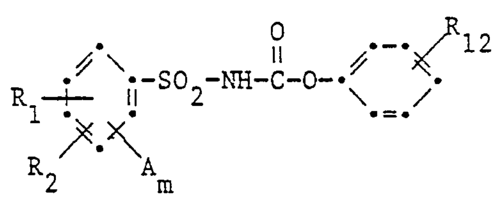

(VII)

worin A, $R_1$, $R_2$ und m die unter Formel I gegebene Bedeutung haben und $R_{12}$ für Wasserstoff, Halogen, $C_1$-$C_4$-Alkyl, $C_1$-$C_4$-Alkoxy, Nitro, Cyan oder Trifluormethyl steht, mit einem Amin der oben angegebenen Formel V umsetzt und gegebenenfalls in ihre Salze überführt, indem man einen Sulfonylharnstoff der Formel I mit einem Amin, einem Alkalimetall- oder Erdalkalimetallhydroxid oder einer quaternären Ammoniumbase umsetzt.

9. Die Verwendung der N-Phenylsulfonyl-N'-triazinyl- oder pyrimidinylharnstoffe der Formel I, Anspruch 1, oder sie enthaltender Mittel zur Bekämpfung unerwünschten Pflanzenwachstums.

10. Die Verwendung der N-Phenylsulfonyl-N'-triazinyl- oder pyrimidinylharnstoffe der Formel I, Anspruch 1, oder sie enthaltender Mittel zur Hemmung des Pflanzenwachstums.

11. Die Verwendung der N-Phenylsulfonyl-N'-triazinyl- oder pyrimidinylharnstoffe der Formel I, oder sie enthaltender Mittel, gemäss Anspruch 9, zur selektiven pre- oder post-emergenten Bekämpfung von Unkräutern in Nutzpflanzenkulturen.

12. Die Verwendung der Verbindungen der Formel I oder sie enthaltender Mittel gemäss Anspruch 10 zur Wuchshemmung von Bodendecker-Leguminosen.

13. Die Verwendung der Verbidungen der Formel I oder sie enthaltender Mittel gemäss Anspruch 10 zur Keimhemmung von Lagerkartoffeln.

14. Die Verwendung der Verbindungen der Formel I oder sie enthaltender Mittel gemäss Anspruch 11 in Getreide, Baumwolle, Soja, Mais und Reis.

**Claims** (for the Contracting States : DE, GB, FR, CH, LI, IT, NL, BE)

1. An N-phenylsulfonyl-N'-pyrimidinylurea or N-phenylsulfonyl-N'-triazinylurea of the general formula I

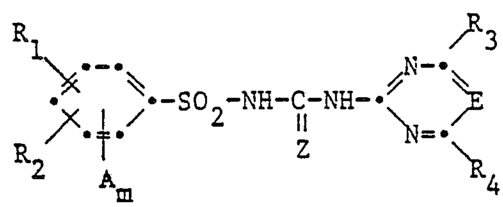

(I)

wherein

A is a radical of the formula —C=C—R,

m is 1 or 2,

E is the methine group or nitogen,

Z is oxygen or sulfur,

R is hydrogen ; branched or unbranched $C_1$-$C_9$-alkyl which is unsubstituted or substituted by halogen, hydroxyl, $C_1$-$C_4$alkoxy, $C_1$-$C_4$alkylthio, $C_1$-$C_4$haloalkoxy, $C_3$-$C_9$cycloalkyl, cyano, $—COOR_6$, $—CONR_7R_8$ or by phenyl which is in turn unsubstituted or substituted by halogen, $C_1$-$C_4$alkyl, $C_1$-$C_4$alkoxy, nitro, cyano or trifluoromethyl ; or is $C_3$-$C_9$cycloalkyl which is unsubstituted or substituted by halogen, hydroxyl, $C_1$-$C_4$alkyl, $C_1$-$C_4$alkoxy, $C_1$-$C_4$alkylthio, cyano., $—COOR_6$, $—CONR_7R_8$ ; or is phenyl or phenyl substituted by halogen, nitro, cyano, $C_1$-$C_4$alkyl, $C_1$-$C_4$alkoxy, $C_1$-$C_4$alkylthio, trifluoromethyl, $—COOR_6$ or $—CONR_7R_8$ ; or is a 5- or 6-membered aromatic heterocyclic ring which is unsubstituted or substituted by halogen, $C_1$-$C_4$alkyl, $C_1$-$C_4$alkoxy, $—COOR_6$ or $—CONR_7R_8$,

$R_1$ is hydrogen, halogen, $C_1$-$C_5$alkyl, $C_2$-$C_5$alkenyl or a radical $—Y—R_5$,

$R_2$ is hydrogen, halogen, $C_1$-$C_5$alkyl, $C_2$-$C_5$alkenyl, $C_1$-$C_4$haloalkyl, or a radical $—Y—R_5$, $—COOR_6$, $—NO_2$ or $—CO—NR_7—R_8$,

$R_3$ and $R_4$, each independently of the other, are hydrogen, $C_1$-$C_4$alkyl, $C_1$-$C_4$alkoxy, $C_1$-$C_4$alkylthio, $C_1$-$C_4$haloalkyl, $C_1$-$C_4$haloalkoxy, $C_1$-$C_4$haloalkylthio, halogen, $C_2$-$C_5$alkoxyalkyl, $—NR_9R_{10}$ or $—O—CH_2—CH_2—NR_9R_{10}$,

$R_5$ and $R_6$, each independently of the other, are $C_1$-$C_5$alkyl, $C_2$-$C_5$alkenyl or $C_2$-$C_6$alkynyl, $C_1$-$C_5$haloalkyl, $C_2$-$C_5$haloalkenyl or $C_2$-$C_6$alkoxyalkyl,

$R_7$ and $R_8$, each independenly of the other, are hydrogen, $C_1$-$C_5$alkyl, $C_2$-$C_5$alkenyl or $C_2$-$C_6$alkynyl,

$R_9$ is hydrogen, methyl or ethyl,

$R_{10}$ is hydrogen, $C_1$-$C_4$alkyl, $C_1$-$C_4$alkoxy, methoxymethyl, cyanomethyl, cyanoethyl, $C_3$-$C_5$alkenyl or $C_3$-$C_6$ alkynyl, and

Y is oxygen, sulfur, a sulfinyl or sulfonyl bridge,

or a salt thereof.

2. A compound according to claim 1, wherein Z is oxygen and m is 1, and $R_3$ and $R_4$ together contain not more than 4 carbon atoms.

3. A compound according to claim 2, wherein only one radical A is in the 2- or 3-position to the sulfonyl group, $R_3$ and $R_4$ together contain not more than 4 carbon atoms, $R_3$ and $R_4$ together contain not more than 4 carbon atoms, $R_1$ is hydrogen and $R_2$ is in the 5- or 6-position to the sulfonyl group.

4. A compound according to claim 3, wherein $R_2$ is hydrogen, chlorine, fluorine, methyl, nitro, trifluoromethyl, $C_1$-$C_4$haloalkoxy, $C_1$-$C_4$alkoxycarbonyl or $C_1$-$C_4$alkoxy, each of $R_3$ and $R_4$ is hydrogen, $C_1$-$C_4$alkyl, $C_1$-$C_4$alkoxy, $C_1$-$C_4$alkylthio, dialkylamino, $C_1$-$C_4$haloalkoxy, halogen or alkoxyalkyl, and $R_3$ and $R_4$ together contain not more than 4 carbon atoms, R is hydrogen or branched or unbranched $C_1$-$C_4$alkyl which is unsubstituted or substituted by halogen, hydroxyl, methoxy, methylthio, $C_1$-$C_4$haloalkoxy, cyclopropyl, cyano, methoxycarbonyl, or is phenyl, pyridyl, thienyl or furyl ; and R is in the 2- or 3-position, and $R_2$ is in the 6-position, to the sulfonyl group.

5. N-[2-(propyn-1-yl)phenylsulfonyl]-N'-(4,6-dimethoxypyrimidin-2-yl)-urea according to claim 1.

6. N-[2-(propyn-1-yl)-6-chlorophenylsulfonyl]-N'-(4-methoxy-6-methyltriazin-2-yl)urea according to claim 1.

7. N-[2-(propion-1-yl)phenylsulfonyl]-N'-(4-methoxy-6-methylpyrimidin-2-yl)urea according to claim 1.

8. A process for the preparation of a compound of the formula I according to claim 1, which comprises either

a) reacting a phenylsulfonamide of the formula II

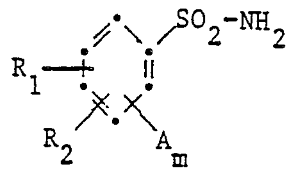

(II)

wherein A, $R_1$, $R_2$ and m are as defined for formula I, with an N-pyrimidinylcarbamate or N-triazinylcarbamate of the formula

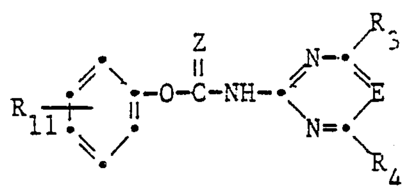

(III)

wherein E, $R_3$, $R_4$ and Z are as defined for formula I and $R_{11}$ is hydrogen, halogen, $C_1$-$C_4$alkyl, $C_1$-$C_4$alkoxy, nitro, cyano or trifluoromethyl, in the presence of a base, or

b) reacting a phenylsulfonylisocyanate or phenylsulfonylisothiocyanate of the formula IV

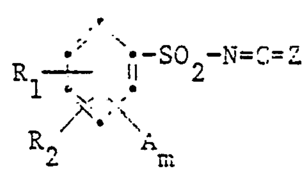

(IV)

wherein A, $R_1$, $R_2$, m and Z are as defined for formula I, with an amine of the formula V

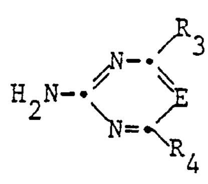

(V)

wherein R, $R_3$ and $R_4$ are as defined for formula I, optionally in the presence of a base, or

c) reacting a sulfonamide of the formula II above with an isocyanate or isothiocyanate of the formula VI

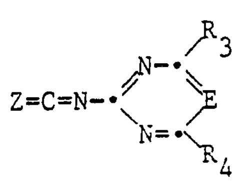

(VI)

wherein E, $R_3$, $R_4$ and Z are as defined for formula I, optionally in the presence of a base, or

d) reacting an N-phenylsulfonylcarbamate of the formula VII

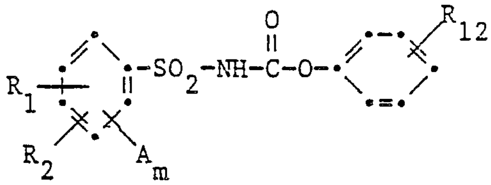

(VII)

wherein A, $R_1$, $R_2$ and m are as defined for formula I and $R_{12}$ is hydrogen, halogen, $C_1$-$C_4$alkyl, $C_1$-$C_4$alkoxy, nitro, cyano or trifluoromethyl, with an amine of the formula V above, and, if desired, converting the sulfonylurea of the formula I into a salt thereof by reaction with an amine, an alkali metal hydroxide or alkaline earth metal hydroxide, or with a quaternary ammonium base.

9. A phenylsulfonamide of the formula II

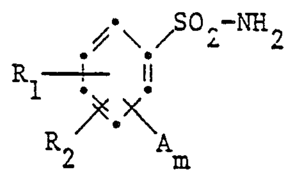

(II)

wherein A, $R_1$, $R_2$ and m are as defined for formula I in claim 1.

10. A herbicidal and plant growth-inhibiting composition which contains at least one N-phenylsulfonyl-N'-triazinylurea or N-phenylsulfonyl-N'-pyrimidinylurea of the formula I according to claim 1, together with carriers and/or other adjuvants.

11. A method of controlling undesired plant growth, which comprises the use of an N-phenylsulfonyl-N'-triazinylurea or N-phenylsulfonyl-N'-pyrimidinylurea of the formula I according to claim 1, or of a composition containing such a compound.

12. A method of inhibiting plant growth, which comprises the use of an N-phenylsulfonyl-N'-triazinylurea or N-phenylsulfonyl-N'-pyrimidinylurea of the formula I according to claim 1, or of a composition containing such a compound.

13. A method according to claim 11 of selectively controlling weeds pre- or postemergence in crops of useful plants, which method comprises the use of an N-phenylsulfonyl-N'-triazinylurea or N-phenylsulfonyl-N'-pyrimidinylurea of the formula I, or of a composition containing such a compound.

14. A method according to claim 12 of inhibiting the growth of leguminous cover crops, which comprises the use of a compound of the formula I, or of a composition containing such a compound.

15. A method according to claim 12 of inhibiting the sprouting of stored potatoes, which comprises the use of a compound of the formula I, or of a composition containing such a compound.

16. A method according to claim 13, wherein the crops of useful plants are cereals, cotton, soybeans, maize and rice.

**Claims** (for the Contracting State AT)

1. A herbicidal and plant growth-inhibiting composition which contains, as active ingredient, at least one N-phenylsulfonyl-N'-triazinyl or N-phenylsulfonyl-N'-pyrimidinylureas of the formula I

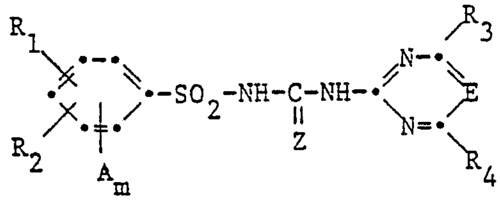

(I)

wherein

A is a radical of the formula —C=C—R,

m is 1 or 2,

E is the methine group or nitrogen,

Z is oxygen or sulfur,

R is hydrogen; branched or unbranched $C_1$-$C_9$alkyl which is unsubstituted or substituted by halogen, hydroxyl, $C_1$-$C_4$alkoxy, $C_1$-$C_4$alkylthio, $C_1$-$C_4$haloalkoxy, $C_3$-$C_9$cycloalkyl, cyano, —$COOR_6$,

—$CONR_7R_8$ or by phenyl which is in turn unsubstituted or substituted by halogen, $C_1$-$C_4$alkyl, $C_{14}$-$C_4$alkoxy, nitro, cyano or trifluoromethyl ; or is $C_3$-$C_9$cycloalkyl which is unsubstituted or substituted by halogen, hydroxyl, $C_1$-$C_4$alkyl, $C_1$-$C_4$alkoxy, $C_1$-$C_4$alkylthio, cyano, —$COOR_6$, —$CONR_7R_8$ ; or is phenyl or phenyl substituted by halogen, nitro, cyano, $C_1$-$C_4$alkyl, $C_1$-$C_4$alkoxy, $C_1$-$C_4$alkylthio, trifluoromethyl, —$COOR_6$ or —$CONR_7R_8$ ; or is a 5- or 6-membered aromatic heterocyclic ring which is unsubstituted or substituted by halogen, $C_1$-$C_4$alkyl, $C_1$-$C_4$alkoxy, —$COOR_6$ or —$CONR_7R_8$,

$R_1$ is hydrogen, halogen, $C_1$-$C_5$alkyl, $C_2$-$C_5$alkenyl or a radical —Y—$R_5$,

$R_2$ is hydrogen, halogen, $C_1$-$C_5$alkyl, $C_2$-$C_5$alkenyl, $C_1$-$C_4$haloalkyl, or a radical —Y—$R_5$, —$COOR_6$, —$NO_2$ or —CO—$NR_7$—$R_8$,

$R_3$ and $R_4$, each independently of the other, are hydrogen, $C_1$-$C_4$alkyl, $C_1$-$C_4$alkoxy, $C_1$-$C_4$alkylthio, $C_1$-$C_4$haloalkyl, $C_1$-$C_4$haloalkoxy, $C_1$-$C_4$haloalkylthio, halogen, $C_2$-$C_5$alkoxyalkyl, —$NR_9R_{10}$ or —O—$CH_2$—$CH_2$—$NR_9R_{10}$,

$R_5$ and $R_6$, each independently of the other, are $C_1$-$C_5$alkyl, $C_2$-$C_5$alkenyl or $C_2$-$C_6$alkenyl, $C_1$-$C_5$haloalkyl, $C_2$-$C_5$haloalkenyl or $C_2$-$C_6$alkoxyalkyl,

$R_7$ and $R_8$, each independently of the other, are hydrogen, $C_1$-$C_5$alkyl, $C_2$-$C_5$alkenyl or $C_2$-$C_6$alkynyl,

$R_9$ is hydrogen, methyl or ethyl,

$R_{10}$ is hydrogen, $C_1$-$C_4$alkyl, $C_1$-$C_4$alkoxy, methoxymethyl, cyanomethyl, cyanoethyl, $C_3$-$C_5$alkenyl or $C_3$-$C_6$alkynyl, and

Y is oxygen, sulfur, a sulfinyl or sulfonyl bridge,

or a salt thereof.

2. A composition according to claim 1, wherein Z is oxygen and m is 1, and $R_3$ and $R_4$ together contain not more than 4 carbon atoms.

3. A composition according to claim 2, wherein only one radical A is an the 2- or 3-position to the sulfonyl group, $R_3$ and $R_4$ together contain not more than 4 carbon atoms, $R_1$ is hydrogen and $R_2$ is in the 5- or 6-position to the sulfonyl group.

4. A composition according to claim 3, wherein $R_2$ is hydrogen, chlorine, fluorine, methyl, nitro, trifluoromethyl, $C_1$-$C_4$haloalkoxy, $C_1$-$C_4$alkoxycarbonyl or $C_1$-$C_4$alkoxy, each of $R_3$ and $R_4$ is hydrogen, $C_1$-$C_4$alkyl, $C_1$-$C_4$alkoxy, $C_1$-$C_4$alkylthio, dialkylamino, $C_1$-$C_3$haloalkoxy, halogen or alkoxyalkyl, and $R_3$ and $R_4$ together contain not more than 4 carbon atoms, R is hydrogen or branched or unbranched $C_1$-$C_4$alkyl which is unsubstituted or substituted by halogen, hydroxyl, methoxy, methylthio, $C_1$-$C_4$haloalkoxy, cyclopropyl, cyano, methoxycarbonyl, or is phenyl, pyridyl, thienyl or furyl ; and R is in the 2- or 3-position, and $R_2$ is in the 6-position, to the sulfonyl group.

5. A composition according to claim 1, which contains, as active ingredient, N-[2-(propyn-1-yl)phenylsulfonyl]-N'-(4,6-dimethoxypyrimidin-2-yl)-urea.

6. A composition according to claim 1, which contains, as active ingredient, N-[2-propyn-1-yl)-6-chlorophenylsulfonyl]-N'-(4-methoxy-6-methyltriazin-2-yl)urea.

7. A composition according to claim 1, which contains, as active ingredient, N-[2-(propion-1-yl)phenylsulfonyl]-N'-(4-methoxy-6-methylpyrimidin-2-yl)urea.

8. A process for the preparation of a compound of the formula I according to claim 1, which comprises either

a) reacting a phenylsulfonamide of the formula II

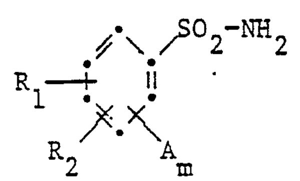

(II)

wherein A, $R_1$, $R_2$ and m are as defined for formula I, with an N-pyrimidinylcarbamate or N-triazinylcarbamate of the formula

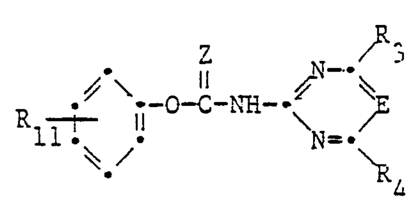

(III)

wherein E, $R_3$, $R_4$ and Z are as defined for formula I and $R_{11}$ is hydrogen, halogen, $C_1$-$C_4$alkyl, $C_1$-$C_4$alkoxy, nitro, cyano or trifluoromethyl, in the presence of a base, or

b) reacting a phenylsulfonylisocyanate or phenylsulfonylisothiocyanate of the formula IV

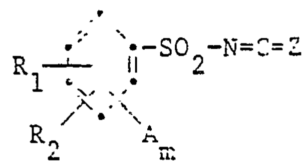

(IV)

wherein A, $R_1$, $R_2$, m and Z are as defined for formula I, with an amine of the formula V

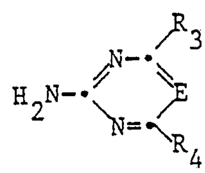

(V)

wherein R, $R_3$ and $R_4$ are as defined for formula I, optionally in the presence of a base, or

c) reacting a sulfonamide of the formula II above with an isocyanate or isothiocyanate of the formula VI

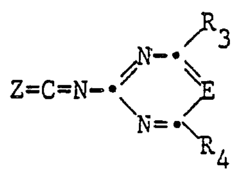

(VI)

wherein E, $R_3$, $R_4$ and Z are as defined for formula I, optionally in the presence of a base, or

d) reacting an N-phenylsulfonylcarbamate of the formula VII

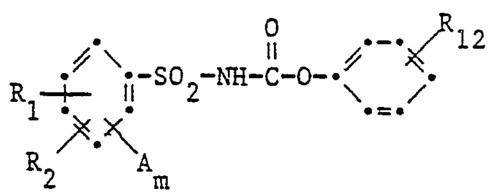

(VII)

wherein A, $R_1$, $R_2$ and m are as defined for formula I and $R_{12}$ is hydrogen, halogen, $C_1$-$C_4$alkyl, $C_1$-$C_4$alkoxy, nitro, cyano or trifluoromethyl, with an amine of the formula V above, and, if desired, converting the sulfonylurea of the formula I into a salt thereof by reaction with an amine, an alkali metal hydroxide or alkaline earth metal hydroxide, or with a quaternary ammonium base.

9. A method of controlling undesired plant growth, which comprises the use of an N-phenylsulfonyl-N'-triazinylurea or N-phenylsulfonyl-N'-pyrimidinylurea of the formula I according to claim 1, or of a composition containing such a compound.

10. A method of inhibiting plant growth, which comprises the use of an N-phenylsulfonyl-N'-triazinylurea or N-phenylsulfonyl-N'-pyrimidinylurea of the formula I according to claim 1, or of a composition containing such a compound.

11. A method according to claim 9 of selectively controlling weeds pre- or postemergence in crops of useful plants, which method comprises the use of an N-phenylsulfonyl-N'-triazinylurea or n-phenylsulfonyl-N'-pyrimidinylurea of the formula I, or of a composition containing such a compound.

12. A method according to claim 10 of inhibiting the growth of leguminous cover crops, which comprises the use of a compound of the formula I, or of a composition containing such a compound.

13. A method according to claim 10 of inhibiting the sprouting of stored potatoes, which comprises the use of a compound of the formula I, or of a composition containing such a compound.

14. A method according to claim 11, wherein the crops of useful plants are cereals, cotton, soybeans, maize and rice.

**Revendications** (pour les Etats contractants : DE, GB, FR, CH, LI, IT, NL, BE)

1. N-phénylsulfonyl-N'-pyrimidinyl- et -triazinylurées de formule générale I

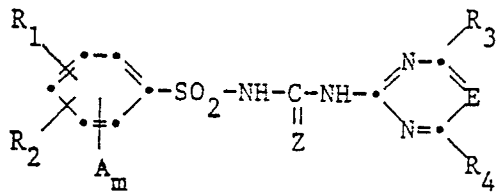

(I)

dans laquelle

A représente un reste de formule —C≡C—R,

m est égal à 1 ou 2,

E représente le groupe méthine ou l'azote,

Z représente l'oxygène ou le soufre,

R représente l'hydrogène ; un groupe alkyle en C1-C9 droit ou ramifié éventuellement substitué par des halogènes, des groupes hydroxy, alcoxy en C1-C4, alkylthio en C1-C4, halogénoalcoxy en C1-C4, cycloalkyle en C3-C9, cyano, $-COOR_6$, $-CONR_7R_8$ ou phényle lui-même éventuellement substitué par des halogènes, des groupes alkyle en C1-C4, alcoxy en C1-C4, nitro, cyano ou trifluorométhyle ; un groupe cycloalkyle en C3-C9 éventuellement substitué par des halogènes, des groupes hydroxy, alkyle en C1-C4, alcoxy en C1-C4, alkylthio en C1-C4, cyano, $-COOR_6$, $-CONR_7R_8$ ; un groupe phényle éventuellement substitué par des halogènes, des groupes nitro, cyano, alkyle en C1-C4, alcoxy en C1-C4, alkylthio en C1-C4, trifluorométhyle, $-COOR_6$ ou $-CONR_7R_8$ ; ou un hétérocycle aromatique à 5 ou 6 chaînons éventuellement substitué par des halogènes, des groupes alkyles en C1-C4, alcoxy en C1-C4, $-COOR_6$ ou $-CONR_7R_8$,

$R_1$ représente l'hydrogène, un halogène, un groupe alkyle en C1-C5, alcényle en C2-C5 ou un reste $-Y-R_5$,

$R_2$ représente l'hydrogène, un halogène, un groupe alkyle en C1-C5, alcényle en C2-C5, halogénoalkyle en C1-C4 ou un reste $-Y-R_5$, $-COOR_6$, $-NO_2$ ou $-CO-NR_7-R_8$,

$R_3$ et $R_4$ représentent chacun, indépendamment l'un de l'autre, l'hydrogène, un groupe alkyle en C1-C4, alcoxy en C1-C4, alkylthio en C1-C4, halogénoalkyle en C1-C4, halogénoalcoxy en C1-C4, halogénoalkylthio en C1-C4, un halogène, un groupe alcoxyalkyle en C2-C5, $-NR_9R_{10}$ ou $-O-CH_2-CH_2-NR_9R_{10}$

$R_5$ et $R_6$ représentent chacun, indépendamment l'un de l'autre, un groupe alkyle en C1-C5, alcényle en C2-C5 ou alcynyle en C2-C6, halogénoalkyle en C1-C5, halogénoalcényle en C2-C5 ou alcoxyalkyle en C2-C6,

$R_7$ et $R_8$ représentent chacun, indépendamment l'un de l'autre, l'hydrogène, un groupe alkyle en C1-C5, alcényle en C2-C5 ou alcynyle en C2-C6,

$R_9$ représente l'hydrogène, un groupe méthyle ou éthyle,

$R_{10}$ représente l'hydrogène, un groupe alkyle en C1-C4, alcoxy en C1-C4, méthoxyméthyle, cyanométhyle, cyaméthyle, alcényle en C3-C5 ou alcynyle en C3-C6 et

Y représente l'oxygène, le soufre, un pont sulfinyle ou sulfonyle,

et les sels de ces composés.

2. Composés selon la revendication 1, caractérisés en ce que Z représente l'oxygène et m est égal à 1, et en ce que les restes $R_3$ et $R_4$, ensemble, ne contiennent pas plus de 4 atomes de carbone.

3. Composés selon la revendication 2, caractérisés en ce qu'il n'y a qu'un seul reste A en position 2 ou 3 par rapport au groupe sulfonyle, les restes $R_3$ et $R_4$, ensemble, ne contiennent pas plus de 4 atomes de carbone, $R_1$ représente l'hydrogène et le reste $R_2$ est en position 5 ou 6 par rapport au groupe sulfonyle.

4. Composés selon la revendication 3, caractérisés en ce que $R_2$ représente l'hydrogène, le chlore, le fluor, un groupe méthyle, nitro, trifluorométhyle, halogénoalcoxy en C1-C4, (alcoxy en C1-C4)-carbonyle ou alcoxy en C1-C4 et $R_3$ et $R_4$ représentent chacun l'hydrogène, un groupe alkyle en C1-C4, alcoxy en C1-C4, alkylthio en C1-C4, dialkylamino, halogénoalcoxy en C1-C3, un halogène ou un groupe alcoxyalkyle, $R_3$ et $R_4$, ensemble, contenant au maximum 4 atomes de carbone, et R représente l'hydrogène ou un groupe alkyle en C1-C4 droit ou ramifié éventuellement substitué par des halogènes, des groupes hydroxy, méthoxy, méthylthio, halogénoalcoxy en C1-C4, cyclopropyle, cyano, méthoxycarbonyle, ou un groupe phényle, pyridyle, thiényle ou furyle, le reste R est en position 2 ou 3 et le reste $R_2$ en position 6 par rapport au groupe sulfonyle.

5. La N-[2-(1-propynyl)-phénylsulfonyl]-N'-(4,6-diméthoxy-pyrimidine-2-yl)-urée selon la revendication 1.

6. La N-[2-(1-propynyl)-6-chloro-phénylsulfonyl]-N'-(4-methoxy-6-méthyl-triazine-2-yl)-urée selon la revendication 1.

7. La N-[2-(1-propionyl)-phénylsulfonyl]-N'-(4-méthoxy-6-méthyl-pyrimidine-2-yl)-urée selon la revendication 1.

8. Procédé de préparation du composé de formule I, revendication 1, caractérisé en ce que :

a) ou bien on fait réagir un phénylsulfonamide de formule II

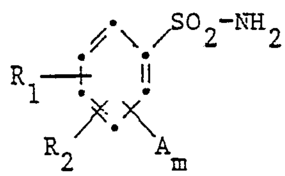

(II)

dans laquelle A, $R_1$, $R_2$ et m ont les significations indiquées en référence à la formule I, en présence d'une base, avec un N-pyrimidinyl- ou -triazinyl-carbamate de formule

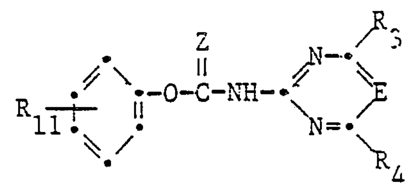

(III)

dans laquelle E, $R_3$, $R_4$ et Z ont les significations indiquées en référence à la formule I et $R_{11}$ représente l'hydrogène, un halogène, un groupe alkyle en C1-C4, alcoxy en C1-C4, nitro, cyano ou trifluorométhyle,

b) ou bien on fait réagir un phénylsulfonylisocyanate ou -isothiocyanate de formule IV

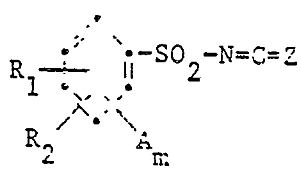

(IV)

dans laquelle A, $R_1$, $R_2$, m et Z ont les significations indiquées en référence à la formule I, éventuellement en présence d'une base, avec une amine de formule V

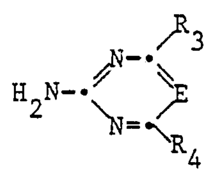

(V)

dans laquelle R, $R_3$ et $R_4$ ont les significations indiquées en référence à la formule I,

c) ou bien on fait réagir des sulfonamides de formule II ci-dessus, éventuellement en présence d'une base, avec un isocyanate ou isothiocyanate de formule VI

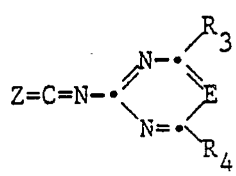

(VI)

dans laquelle E, $R_3$, $R_4$ et Z ont les significations indiquées en référence à la formule I,

d) ou bien on fait réagir un N-phénylsulfonylcarbamate de formule VII

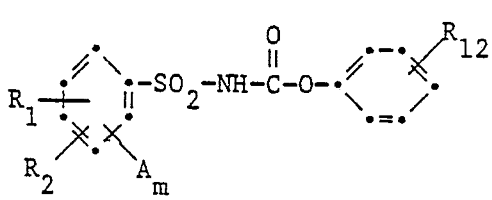

(VII)

dans laquelle A, $R_1$, $R_2$ et m ont les significations indiquées en référence à la formule I et $R_{12}$ représente l'hydrogène, un halogène, un groupe alkyle en C1-C4, alcoxy en C1-C4, nitro, cyano ou trifluorométhyle, avec une amine de formule V ci-dessus et le cas échéant on convertit en les sels en faisant réagir une sulfonylurée de formule I avec une amine, un hydroxyde de métal alcalin ou alcalino-terreux ou une base d'ammonium quaternaire.

9. Phénylsulfonamides de formule II

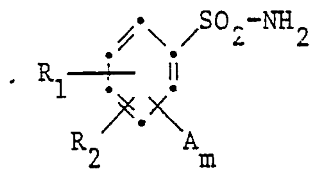

(II)

dans laquelle A, $R_1$, $R_2$ et m ont les significations indiquées en référence à la formule I, revendication 1.

10. Un produit herbicide et inhibiteur de la croissance des végétaux, caractérisé en ce qu'il contient en tant que substance active, avec des véhicules et/ou d'autres additifs, au moins une N-phénylsulfonyl-N'-triazinyl- ou -pyrimidinyl-urée de formule I, revendication 1.

11. L'utilisation des N-phénylsulfonyl-N'-triazinyl- ou -pyrimidinyl-urées de formule I, revendication 1, ou de produits en contenant dans la lutte contre les croissances de végétaux indésirables.

12. L'utilisation des N-phénylsulfonyl-N'-triazinyl- ou -pyrimidinyl-urées de formule I, revendication 1, ou de produits en contenant pour l'inhibition de la croissance des végétaux.

13. L'utilisation des N-phénylsulfonyl-N'-triazinyl- ou -pyrimidinyl-urées de formule I, ou de produits en contenant, selon la revendication 11, pour la lutte sélective en pré-levée ou en post-levée contre les mauvaises herbes dans les cultures de végétaux utiles.

14. L'utilisation des composés de formule I ou de produits en contenant selon la revendication 12, pour l'inhibition de la croissance des légumineuses de couvertures de sols.

15. L'utilisation des composés de formule I ou de produits en contenant selon la revendication 12, pour l'inhibition de la germination des pommes de terre stockées.

16. L'utilisation des composés de formule I ou de produits en contenant selon la revendication 13, dans les cultures de céréales, de coton, de soja, de maïs et de riz.

**Revendications** (pour l'Etat contractant AT)

1. Un produit herbicide et inhibiteur de la croissance des végétaux caractérisé en ce qu'il contient en tant que substance active, avec des véhicules et/ou d'autres additifs, au moins une N-phénylsulfonyl-N'-triazinyl- ou -pyrimidinyl-urée de formule I

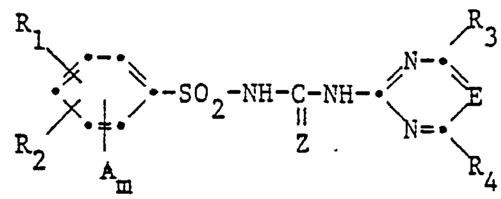

(I)

ou ses sels, dans lesquels

A représente un reste de formule —C≡C—R,

m est égal à 1 ou 2,

E représente le groupe méthine ou l'azote,

Z représente l'oxygène ou le soufre,

R représente l'hydrogène ; un groupe alkyle en C1-C9 droit ou ramifié éventuellement substitué par des halogènes, des groupes hydroxy, alcoxy en C1-C4, alkylthio en C1-C4, halogénoalcoxy en C1-C4, cycloalkyle en C3-C9, cyano, $—COOR_6$, $—CONR_7R_8$ ou phényle lui-même éventuellement substitué par des halogènes, des groupes alkyle en C1-C4, alcoxy en C1-C4, nitro, cyano ou trifluorométhyle ; un groupe cycloalkyle en C3-C9 éventuellement substitué par des halogènes, des groupes hydroxy, alkyle en C1-C4, alcoxy en C1-C4, alkylthio en C1-C4, cyano, $—COOR_6$, $—CONR_7R_8$ ; un groupe phényle éventuellement substitué par des halogènes, des groupes nitro, cyano, alkyle en C1-C4, alcoxy en C1-C4, alkylthio en C1-C4, trifluorométhyle, $—COOR_6$ ou $—CONR_7R_8$ ; ou un hétérocycle aromatique à 5 ou 6 chaînons éventuellement substitué par des halogènes, des groupes alkyle en C1-C4, alcoxy en C1-C4, $—COOR_6$ ou $—CONR_7R_8$,

$R_1$ représente l'hydrogène, un halogène, un groupe alkyle en C1-C5, alcényle en C2-C5 ou un reste $—Y—R_5$,

$R_2$ représente l'hydrogène, un halogène, un groupe alkyle en C1-C5, alcényle en C2-C5, halogénoalkyle en C1-C4 ou un reste $—Y—R_5$, $—COOR_6$, $—NO_2$ ou $—CO—NR7—R_8$,

$R_3$ et $R_4$ représentent chacun, indépendamment l'un de l'autre, l'hydrogène, un groupe alkyle en C1-C4, alcoxy en C1-C4, alkylthio en C1-C4, halogénoalkyle en C1-C4, halogénoalcoxy en C1-C4, halogénoalkylthio en C1-C4, un halogène, un groupe alcoxyalkyle en C2-C5, $—NR_9R_{10}$ ou $—O—CH_2—CH_2—NR_9R_{10}$

$R_5$ et $R_6$ représentent chacun, indépendamment l'un de l'autre, un groupe alkyle en C1-C5, alcényle en C2-C5 ou alcynyle en C2-C6, halogénoalkyle en C1-C5, halogénoalcényle en C2-C5 ou alcoxy-alkyle en C2-C6,

$R_7$ et $R_8$ représentent chacun, indépendamment l'un de l'autre, l'hydrogène, un groupe alkyle en C1-C5, alcényle en C2-C5 ou alcynyle en C2-C6,

$R_9$ représente l'hydrogène, un groupe méthyle ou éthyle,

$R_{10}$ représente l'hydrogène, un groupe alkyle en C1-C4, alcoxy en C1-C4, méthoxyméthyle, cyanométhyle, cyanéthyle, alcényle en C3-C5 ou alcynyle en C3-C6 et

Y représente l'oxygène, le soufre, un pont sulfinyle ou sulfonyle.

2. Produit selon la revendication 1, caractérisé en ce que Z représente l'oxygène et m est égal à 1 et en ce que les restes $R_3$ et $R_4$, ensemble, ne contiennent pas plus de 4 atomes de carbone.

3. Produit selon la revendication 2, caractérisé en ce qu'il n'y a qu'un seul reste A en position 2 ou 3 par rapport au groupe sulfonyle, les restes $R_3$ et $R_4$, ensemble, ne contiennent pas plus de 4 atomes de carbone, $R_1$ représente l'hydrogène et le reste $R_2$ est en position 5 ou 6 par rapport au groupe sulfonyle.

4. Produit selon la revendication 3, caractérisé en ce que $R_2$ représente l'hydrogène, le chlore, le fluor, un groupe méthyle, nitro, trifluorométhyle, halogénoalcoxy en C1-C4, (alcoxy en C1-C4)-carbonyle ou alcoxy en C1-C4 et $R_3$ et $R_4$ représentent chacun l'hydrogène, un groupe alkyle en C1-C4, alcoxy en C1-C4, alkylthio en C1-C4, dialkylamino, halogénoalcoxy en C1-C3, un halogène ou un groupe alcoxyalkyle, $R_3$ et $R_4$, ensemble, ne contiennent pas plus de 4 atomes de carbone et R représente l'hydrogène ou un groupe alkyle en C1-C4 droit ou ramifié éventuellement substitué par des halogènes, des groupes hydroxy, méthoxy, méthylthio, halogénoalcoxy en C1-C4, cyclopropyle, cyano, méthoxy-carbonyle, ou un groupe phényle, pyridyle, thiényle ou furyle, le reste R est en position 2 ou 3 et le reste $R_2$ en position 6 par rapport au groupe sulfonyle.

5. Produit selon la revendication 1, caractérisé en ce qu'il contient en tant que substance active la N-[2-(1-propynyl)-phénylsulfonyl]-N'-(4,6-diméthoxy-pyrimidine-2-yl)-urée.

6. Produit selon la revendication 1, caractérisé en ce qu'il contient en tant que substance active la N-[2-(1-propynyl)-6-chloro-phénylsulfonyl]-N'-(4-méthoxy-6-méthyl-triazine-2-yl)-urée.

7. Produit selon la revendication 1, caractérisé en ce qu'il contient en tant que substance active la N-[2-(1-propionyl)-phénylsulfonyl]-N'-(4-méthoxy-6-méthyl-pyrimidine-2-yl)-urée.

8. Procédé de préparation des composés de formule I, selon la revendication 1, caractérisé en ce que :

a) ou bien on fait réagir un phénylsulfonamide de formule II

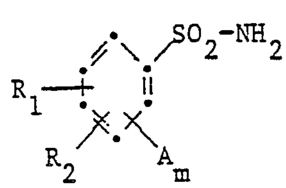

(II)

dans laquelle A, $R_1$, $R_2$ et m ont les significations indiquées en référence à la formule I, en présence d'une base, avec un N-pyrimidinyl- ou -triazinyl-carbamate de formule

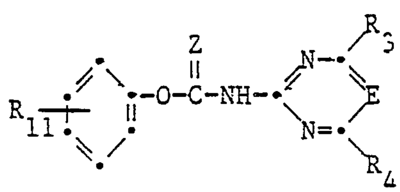

(III)

dans laquelle E, $R_3$, $R_4$ et Z ont les significations indiquées en référence à la formule I et $R_{11}$ représente l'hydrogène, un halogène, un groupe alkyle en C1-C4, alcoxy en C1-C4, nitro, cyano ou trifluorométhyle,

b) ou bien on fait réagir un phénylsulfonylisocyanate ou -isothiocyanate de formule IV

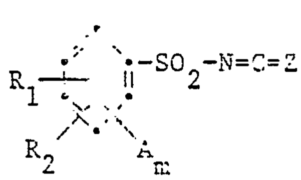

(IV)

dans laquelle A, $R_1$, $R_2$, m et Z ont les significations indiquées en référence à la formule I, éventuellement en présence d'une base, avec une amine de formule V

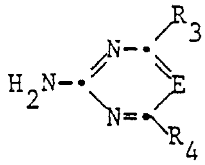

(V)

dans laquelle R, $R_3$ et $R_4$ ont les significations indiquées en référence à la formule I,

c) ou bien on fait réagir des sulfonamides de formule II ci-dessus, éventuellement en présence d'une base, avec un isocyanate ou isothiocyanate de formule VI

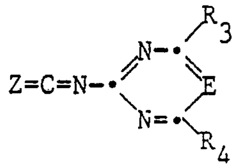

(VI)

dans laquelle E, $R_3$, $R_4$ et Z ont les significations indiquées en référence à la formule I,

d) ou bien on fait réagir un N-phénylsulfonylcarbamate de formule VII

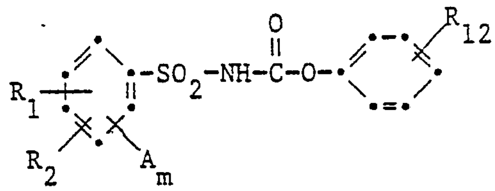

(VII)

dans laquelle A, $R_1$, $R_2$ et m ont les significations indiquées en référence à la formule I et $R_{12}$ représente l'hydrogène, un halogène, un groupe alkyle en C1-C4, alcoxy en C1-C4, nitro, cyano ou trifluorométhyle, avec une amine de formule V ci-dessus et le cas échéant on convertit en les sels en faisant réagir une sulfonylurée de formule I avec une amine, un hydroxyde de métal alcalin ou alcalino-terreux ou une base d'ammonium quaternaire.

9. L'utilisation des N-phénylsulfonyl-N'-triazinyl- ou -pyrimidinyl-urées de formule I, revendication 1, ou de produits en contenant dans la lutte contre les croissances de végétaux indésirables.

10. L'utilisation des N-phénylsulfonyl-N'-triazinyl- ou -pyrimidinyl-urées de formule I, revendication 1, ou de produits en contenant pour l'inhibition de la croissance des végétaux.

11. L'utilisation des N-phénylsulfonyl-N'-triazinyl- ou -pyrimidinyl-urées de formule I, ou de produits en contenant, selon la revendication 9, pour la lutte sélective en pré-levée ou en post-levée contre les mauvaises herbes dans les cultures de végétaux utiles.

12. L'utilisation des composés de formule I ou de produits en contenant selon la revendication 10, pour l'inhibition de la croissance des légumineuses de couvertures de sols.

13. L'utilisation des composés de formule I ou de produits en contenant selon la revendication 10, pour l'inhibition de la germination des pommes de terre stockées.

14. L'utilisation des composés de formule I ou de produits en contenant selon la revendication 11, dans les cultures de céréales, de coton, de soja, de maïs et de riz.